# EUROPEAN PATENT SPECIFICATION

(11) **EP 1 635 276 B1**
(45) Date of publication and mention of the grant of the patent: **27.04.2011**
(21) Application number: 05005281.0
(22) Date of filing: 10.03.2005
(51) Int. Cl.: G06F 19/00

(54) **Display method and display apparatus of gene information**
Verfahren und Vorrichtung zur Anzeige von Geninformation
Méthode et apparéil pour l'affichage d'information sur les gènes

(30) Priority: 09.09.2004 JP 2004262431
(43) Date of publication of application: 15.03.2006
(73) Proprietor: Hitachi Solutions, Ltd., Tokyo 140-0002 (JP)
(72) Inventor: Yukawa, Wataru, 4-12-7, Higashishinagawa Tokyo 140-0002 (JP); Matsumoto, Toshiko, 4-12-7, Higashishinagawa Tokyo 140-0002 (JP); Nakashige, Ryo, 4-12-7, Higashishinagawa Tokyo 140-0002 (JP)
(74) Representative: Liesegang, Roland

(56) References cited:
- US-A1- 2002 116 135
- US-B1- 6 274 317
- PARKER L T ET AL: "PEAK HEIGHT VARIATIONS IN AUTOMATED SEQUENCING OF PCR PRODUCTS USING TAQ DYE-TERMINATOR CHEMISTRY" BIOTECHNIQUES, INFORMA LIFE SCIENCES PUBLISHING, WESTBOROUGH, MA, US, vol. 19, no. 1, 1995, pages 116-121, XP009083290 ISSN: 0736-6205
- MILLER M J YUAN B-Z: "SEMIAUTOMATED RESOLUTION OF OVERLAPPING STUTTER PATTERNS IN GENOMICS MICROSATELLITE ANALYSIS" ANALYTICAL BIOCHEMISTRY, ACADEMIC PRESS, SAN DIEGO, CA, US, vol. 251, 1997, pages 50-56, XP002953989 ISSN: 0003-2697
- JOHANSSON ASA ET AL: "A novel method for automatic genotyping of microsatellite markers based on parametric pattern recognition." HUMAN GENETICS, vol. 113, no. 4, September 2003 (2003-09), pages 316-324, XP002431131 ISSN: 0340-6717

### SEQUENCE LISTING

<110> Hitachi Software Engineering Co., Ltd.
<120> DISPLAY METHOD AND DISPLAY APPARATUS OF GENE INFORMATION
<130> P04-0391
<140>
   <141>
<160> 28
<170> PatentIn Ver. 2. 1
<210> 1
   <211> 69
   <212> DNA
   <213> Artificial Sequence
<220>
   <223> Description of Artificial Sequence:Synthetic DNA
<400> 1
   atgctagcta gctagctagc tagctagcta gctagctagc tagctagcta ctgggggggg 60
   gggggggcg 69
<210> 2
   <211> 33
   <212> DNA
   <213> Artificial Sequence
<220>
   <223> Description of Artificial Sequence:Synthetic DNA
<400> 2
   atnnnnnnnn nnnnctgggg gggggggggg gcg 33
<210> 3
   <211> 69
   <212> DNA
   <213> Artificial Sequence
<220>
   <223> Description of Artificial Sequence:Synthetic DNA
<400> 3
   atgctagcta gctagctagc tagctagcta gctagctagc tagctagcta ctgggggggg 60
   gggggggcg 69
<210> 4
   <211> 113
   <212> DNA
   <213> Artificial Sequence
<220>
   <223> Description of Artificial Sequence:Synthetic DNA
<400> 4
   atgcgactat gctagctagc ggctagctag ctagctagct agctagctag ctagctagct 60
   agctagctag ctagctactg gggggggggg ggggcggcta cgagcttacg agc 113
<210> 5
   <211> 69
   <212> DNA
   <213> Artificial Sequence
<220>
   <223> Description of Artificial Sequence:Synthetic DNA
<400> 5
   atgctagcta gctagctagc tagctagcta gctagctagc tagctagcta ctgggggggg 60
   gggggggcg 69
<210> 6
   <211> 33
   <212> DNA
   <213> Artificial Sequence
<220>
   <223> Description of Artificial Sequence:Synthetic DNA
<400> 6
   atnnnnnnnn nnnnctgggg gggggggggg gcg 33
<210> 7
   <211> 54
   <212> DNA
   <213> Artificial Sequence
<220>
   <223> Description of Artificial Sequence:Synthetic DNA
<400> 7
   atcacacaca cacacacaca cacacaggat atatatatat atatatatat atat 54
<210> 8
   <211> 29
   <212> DNA
   <213> Artificial Sequence
<220>
   <223> Description of Artificial Sequence:Synthetic DNA
<400> 8
   atnnnnnnnn nnnnggmmmm mmmmmmmmm 29
<210> 9
   <211> 10
   <212> DNA
   <213> Artificial Sequence
<220>
   <223> Description of Artificial Sequence:Synthetic DNA
<400> 9
   atatatatat 10
<210> 10
   <211> 14
   <212> DNA
   <213> Artificial Sequence
<220>
   <223> Description of Artificial Sequence:Synthetic DNA
<400> 10
   atatatatat atat 14
<210> 11
   <211> 12
   <212> DNA
   <213> Artificial Sequence
<220>
   <223> Description of Artificial Sequence:Synthetic DNA
<400> 11
   atatatatat at 12
<210> 12
   <211> 66
   <212> DNA
   <213> Artificial Sequence
<220>
   <223> Description of Artificial Sequence:Synthetic DNA
<400> 12
   atgcgactat gcgactgcgc ctatatatat atatatatat atatatcggc tacgagctta 60
   cgagct 66
<210> 13
   <211> 58
   <212> DNA
   <213> Artificial Sequence
<220>
   <223> Description of Artificial Sequence:Synthetic DNA
<400> 13
   atgcgactat gcgactgcgc ctatatatat atatatatcg gctacgagct tacgagct 58
<210> 14
   <211> 66
   <212> DNA
   <213> Artificial Sequence
<220>
   <223> Description of Artificial Sequence:Synthetic DNA
<400> 14
   atgcgactat gcgactgcgc ctatatatat atatatatat atatatcggc tacgagctta 60
   cgagct 66
<210> 15
   <211> 58
   <212> DNA
   <213> Artificial Sequence
<220>
   <223> Description of Artificial Sequence:Synthetic DNA
<400> 15
   atgcgactat gcgactgcgc ctatatatat atatatatcg gctacgagct tacgagct 58
<210> 16
   <211> 66
   <212> DNA
   <213> Artificial Sequence
<220>
   <223> Description of Artificial Sequence:Synthetic DNA
<400> 16
   atgcgactat gcgactgcgc ctatatatat atatatatat atatatcggc tacgagctta 60
   cgagct 66
<210> 17
   <211> 66
   <212> DNA
   <213> Artificial Sequence
<220>
   <223> Description of Artificial Sequence:Synthetic DNA
<400> 17
   atgcgactat gcgactgcgc ctatatatat atatatatat atatatcggc tacgagctta 60
   cgagct 66
<210> 18
   <211> 67
   <212> DNA
   <213> Artificial Sequence
<220>
   <223> Description of Artificial Sequence:Synthetic DNA
<400> 18
   atgcgactat gcgactgcgc ctatatatat atatatatat atatatcggc tacgagctta 60
   cgagcta 67
<210> 19
   <211 64
   <212> DNA
   <213> Artificial Sequence
<220>
   <223> Description of Artificial Sequence:Synthetic DNA
<400> 19
<210> 20
   <211> 65
   <212> DNA
   <213> Artificial Sequence
<220>
   <223> Description of Artificial Sequence:Synthetic DNA
<400> 20
   atgcgactat gcgactgcgc ctatatatat atatatatat atatcggcta cgagcttacg 60
   agcta 65
<210> 21
   <211> 68
   <212> DNA
   <213> Artificial Sequence
<220>
   <223> Description of Artificial Sequence:Synthetic DNA
<400> 21
   atgcgactat gcgactgcgc ctatatatat atatatatat atatatatcg gctacgagct 60
   tacgagct 68
<210> 22
   <211> 69
   <212> DNA
   <213> Artificial Sequence
<220>
   <223> Description of Artificial Sequence:Synthetic DNA
<400> 22
   atgcgactat gcgactgcgc ctatatatat atatatatat atatatatcg gctacgagct 60
   tacgagcta 69
<210> 23
   <211> 69
   <212> DNA
   <213> Artificial Sequence
<220>
   <223> Description of Artificial Sequence:Synthetic DNA
<400> 23
   atgctagcta gctagctagc tagctagcta gctagctagc tagctagcta ctgggggggg 60
   gggggggcg 69
<210> 24
   <211> 65
   <212> DNA
   <213> Artificial Sequence
<220>
   <223> Description of Artificial Sequence:Synthetic DNA
<400> 24
   atgctagcta gctagctagc tagctagcta gctagctagc tagctactgg gggggggggg 60
   gggcg 65
<210> 25
   <211> 68
   <212> DNA
   <213> Artificial Sequence
<220>
   <223> Description of Artificial Sequence:Synthetic DNA
<400> 25
   atgctagcta gctagctagc tagctagcta gctagctagc tagctagcta ctgggggggg 60
   ggggggcg 68
<210> 26
   <211> 84
   <212> DNA
   <213> Artificial Sequence
<220>
   <223> Description of Artificial Sequence:Synthetic DNA
<400> 26
   atgctagcta gctagctagc tagctagcta gctagctagc tagctagcta ctcacacaca 60
   cacacacaca cacacacaca cacg 84
<210> 27
   <211> 80
   <212> DNA
   <213> Artificial Sequence
<220>
   <223> Description of Artificial Sequence:Synthetic DNA
<400> 27
   atgctagcta gctagctagc tagctagcta gctagctagc tagctactca cacacacaca 60
   cacacacaca cacacacacg 80
<210> 28
   <211> 82
   <212> DNA
   <213> Artificial Sequence
<220>
   <223> Description of Artificial Sequence:Synthetic DNA
<400> 28
   atgctagcta gctagctagc tagctagcta gctagctagc tagctagcta ctcacacaca 60
   cacacacaca cacacacaca cg 82

## Description

### FIELD OF THE INVENTION

The present invention relates to a display method and a display apparatus of gene information used for genetic analysis study to identify genes affecting phenotypes such as individual's disease and physical external trait, and particularly to a method and an apparatus that allow signals from an analysis target and noise signals to be accurately discriminated and displayed when a DNA fragment containing a target gene is extracted and detected by PCR, electrophoresis, and the like.

### BACKGROUND OF THE INVENTION

Following the completion of the human genome sequencing, research in the study of gene function analysis is actively pursued. Above all, special attention is attracted to automated genotyping that is fundamental to search for genes affecting phenotypes such as the presence or absence of particular diseases, the differences in drug efficacy, and the presence or absence of drug side effects.

### Microsatellite

Generally, the genome of the same species of an organism is approximately the same in nucleotide sequences, but there are several loci having different nucleotides among individuals. For example, there is a case where one individual has A at a single genetic locus while another individual has T at the same locus. As described, a polymorphism seen for a single nucleotide in the genome among individuals is called single nucleotide polymorphism (SNP).

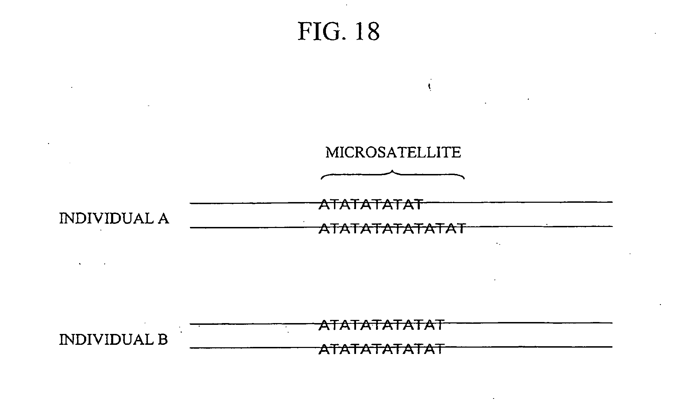

On the other hand, there are many loci (more than several tens of thousand loci) where short sequence pattern of two to six nucleotides is repeated several times to several tens of times to appear in the genome of an organism. This characteristic sequence pattern is called microsatellite. An example of a microsatellite appearing in a genome is shown in Fig. 18. A repeat unit in a microsatellite is referred to as unit, and the number of nucleotides in one unit is referred to as unit length. For example, in the microsatellite of ATATATAT... shown in Fig. 18, the unit is "AT", and the unit length is two nucleotides. The number of repeats in a microsatellite may differ among individuals even if the unit and the unit length are the same among them as shown in Fig. 18.

Since SNP and microsatellite may differ among individuals as described above, it is rather easy to discriminate these loci from other nucleotide sequences and also to detect them experimentally in the genome. Approximate loci of SNPs and microsatellites present in the genome are known for certain species of organisms, and therefore these can be utilized as markers to indicate a genetic locus in the genome. Owing to this nature, SNPs and microsatellites are called DNA markers. Particularly, since microsatellites consist of a plurality of nucleotides and thus contain more information than SNPs, microsatellites are frequently used as DNA markers.

As shown in Fig. 18, an individual of most organisms is provided with a pair of genomes (homologous chromosomes) originating from female gamete and male gamete. Genes located at positions corresponding to each other in the pair of genomes are called alleles, respectively, and the combination of these alleles is called genotype. Since SNPs and microsatellites may represent portions different in nucleotide sequences among individuals as described above, there are two or three alleles for a SNP locus and several to twenty or more kinds of alleles for a microsatellite locus. In an example shown in Fig. 18, an individual A has a pair of microsatellites in which a unit of "AT" is repeated five and seven times, respectively, while an individual B has a pair of microsatellites in which a unit of "AT" is repeated six times, respectively. Herein, the state of having two different alleles as in the case of the individual A is called heterozygosis and the state of having two copies of the same allele as in the case of the individual B is called homozygosis.

### PCR and electrophoresis experiment

When a microsatellite is used as a DNA marker, experiments such as polymerase chain reaction (PCR) and electrophoresis are carried out to extract and detect a locus where the microsatellite appear in the genome. PCR is an experimental technique in which a pair of nucleotide sequences called primer sequence is assigned at both ends of the microsatellite and only the microsatellite portion sandwiched between them is repeatedly replicated as a DNA fragment, yielding a certain amount of a sample. Electrophoresis is an experimental technique in which an amplified DNA fragment is electrophoresed in a charged electrophoresis channel and DNA fragments of different lengths are separated. For electrophoresis, there are methods such as gel electrophoresis and capillary electrophoresis. Electrophoresis is a method for separating a sample by taking advantage of differences in mobility in the electrophoresis channel according to the lengths of DNA fragments (a longer DNA fragment has lower mobility).

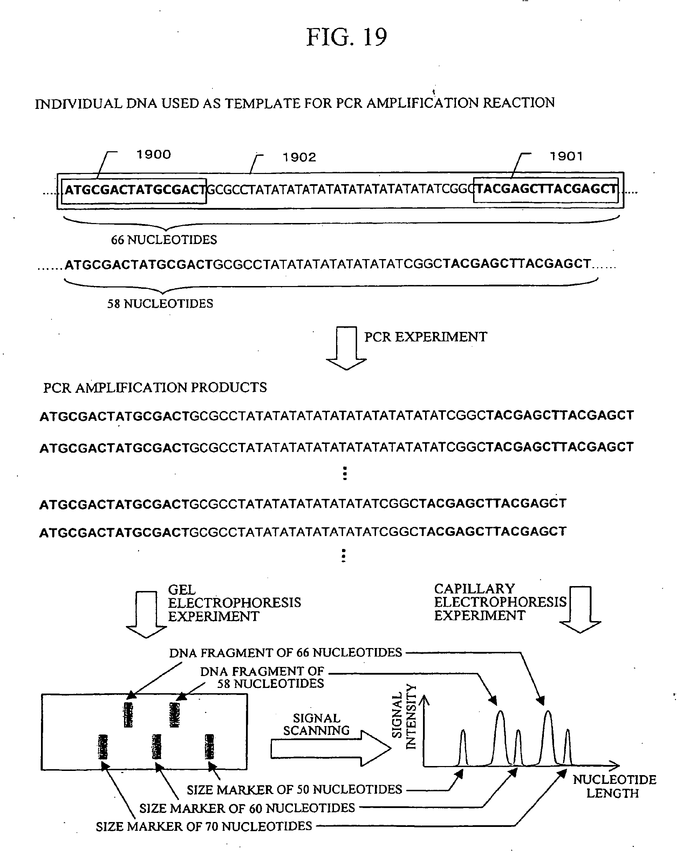

Fig. 19 is a schematic illustration of an experimental procedure to extract and amplify a DNA fragment in a microsatellite portion by PCR and gel electrophoresis. First, a pair of primer sequences 1900 and 1901 that sandwich the target microsatellite are assigned, and the genome region 1902 including the microsatellite and the primer sequence is amplified in a PCR experiment. The example shown in Fig. 19 represents a heterozygote with different numbers of repeats for a microsatellite on two homologous chromosomes. Since the lengths of the microsatellite portions differ from each other, two kinds of PCR amplification products, that is, DNA fragments different in length (66 nucleotides and 58 nucleotides) are obtained from their respective portions. When the above two kinds of PCR amplification products are electrophoresed on a gel plate for a certain time, these are separated according to the difference in length of the DNA fragments. Detection of the position of each DNA fragment, that has been prelabeled with a fluorescent dye, by fluorescence after electrophoresis gives rise to a pattern as shown on the lower left of the illustration in Fig. 19. The length of each PCR product can be determined by running DNA fragments of known lengths (called size markers) with the PCR amplification products in the same electrophoresis and comparing with the detection positions of the size markers.

Although the experimental technique with the use of gel electrophoresis has been described above, capillary electrophoresis can also be used similarly. The capillary electrophoresis is a technique in which a sample is electrophoresed in a narrow tube packed with gel and time required to run past a predetermined distance (generally up to the end of the capillary) is measured for various samples respectively, thereby determining the lengths of DNA fragments. As the result of the capillary electrophoresis, a waveform plot (a group of peaks) with the length of DNA fragment on the horizontal axis versus the signal density on the vertical axis is obtained as shown on the lower right of the illustration in Fig. 19. In general, a sample is detected by a fluorescence signal detector provided at the end portion of the capillary instead of scanning fluorescent signals emitted by the sample in the gel.

### Noises occurring in PCR and electrophoresis experiments

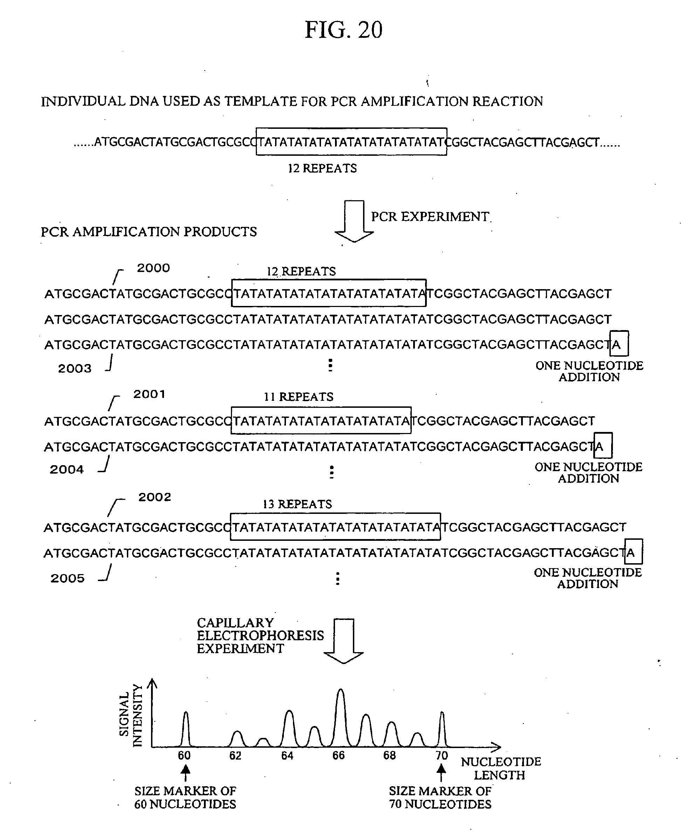

The experimental result shown in Fig. 19 is obtained when PCR and electrophoresis experiments are carried out in an ideal process, while various noises may occur in practical experiments. Representative noises that occur during the course of PCR and electrophoresis experiments, stutter peaks and +A peaks, are explained below with reference to Fig. 20. For simplicity, only the DNA fragment with a length of 66 nucleotides (includes a microsatellite having 12 repeats of "TA") shown in Fig. 19 is exemplified in Fig. 20.

stutter peaks are noises resulting from a phenomenon that the number of repeats in a microsatellite portion of the target DNA fragment to be replicated increases or decreases due to slipped-strand mispairing (occurrence of slipping of the repeat portion of microsatellite) that occurs during PCR reaction. DNA fragments with increased or decreased numbers of repeats are observed as noise peaks in the fluorescence analysis. As shown in Fig. 20, DNA fragments 2001 and 2002 containing abnormal microsatellites with 11 repeats and 13 repeats of "TA" respectively are generated in addition to the DNA fragment 2000 containing the normal microsatellite with 12 repeats of "TA", and these are observed as stutter peaks in the fluorescence analysis. Since a further increase or decrease in the number of repeats may sometimes occur, it is possible that, in addition to the DNA fragment (66 nucleotides) with the same length as that of the DNA fragment used originally for replication, DNA fragments with increased or decreased lengths by integral multiples of the unit length of the microsatellite are generated by carrying out PCR.

+A peaks are noises resulting from a phenomenon that an extra nucleotide (generally A) is added to a DNA fragment during replicating the DNA fragment by PCR, and the DNA fragment with an additional nucleotide is observed as a noise peak in the fluorescence analysis. As shown in Fig. 20, a DNA fragment 2003 with an extra nucleotide added to the normally replicated DNA fragment 2000 is generated, and further, an additional nucleotide is sometimes added to the abnormal DNA fragments 2001 and 2002 with decreased and increased number of repeats respectively in the microsatellite portion due to slipped-strand mispairing to generate DNA fragments 2004 and 2005, respectively. These DNA fragments with an added nucleotide 2003, 2004, and 2005 are observed as each different +A peak in the fluorescence analysis.

In the graph of Fig. 20 showing a result of the fluorescence analysis, a peak arising from the DNA fragment of 66 nucleotides having the same length as that of the DNA fragment originally used for replication is the peak to be primarily observed (hereinafter, referred to as true peak), and peaks other than that are all noise peaks. It is found that stutter peaks appear at positions (positions of 62, 64, and 68 nucleotides) distant from each other by the unit length of the microsatellite with respect to the true peak. Further, it is found that +A peaks appear at positions longer by one nucleotide than each of the true peak and the stutter peaks (positions of 63, 65, 67, and 69 nucleotides). That is, the +A peaks appearing at the positions of 63, 65, 67, and 69 nucleotides correspond to the DNA fragments in which one nucleotide was added to the DNA fragments with 62, 64, 66 and 68 nucleotide lengths, respectively. Hereinafter, the true peak or the stutter peak corresponding to the DNA fragment without an added nucleotide from which a certain +A peak originates is called "original peak" relative to the +A peak.

In the course of PCR and electrophoresis experiments, it is very important to discriminate the true peak and other noises among a plurality of peaks observed in the fluorescence analysis. As to the two kinds of noise peaks, stutter peaks and +A peaks described above, the cause leading to such peaks has been widely studied from molecular biology, and studies on characteristics of their peak heights have also been carried out. These studies resulted in the development of various methods to judge and remove stutter peaks and +A peaks automatically based on waveform data of the fluorescence analysis result.

As a first method, there is a technique in which the highest peak in the waveform data is regarded as the true peak and peaks located at positions distant from the true peak by several nucleotides (specified by a user) are judged to be noise peaks (stutter peaks and +A peaks) and discarded. For example, ABI software "Genotyper" (PerkinElmer, Inc.) employs this method.

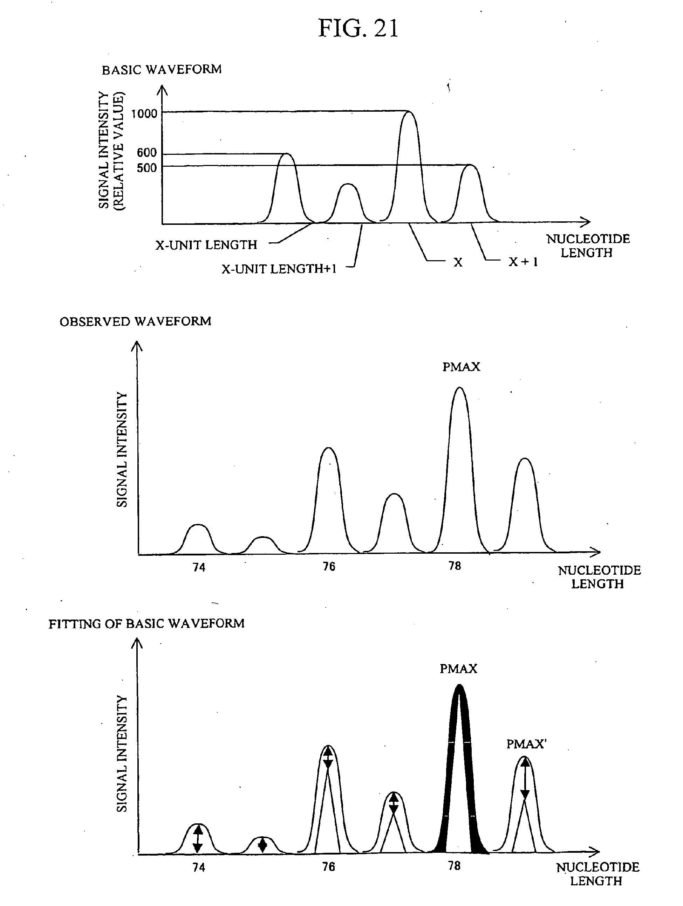

As a second method, there is a technique in which the way noise peaks (stutter peaks and +A peaks) emerge is made in a model for every marker and for every individual, thereby performing peak judgment. This method is explained with reference to Fig. 21. In the upper row of Fig. 21, a waveform model (hereinafter, called basic waveform) including a true peak, its corresponding stutter peaks, and further +A peaks corresponding to these peaks is shown. What is modeled here is a relative height (signal intensity) of each stutter peak and +A peak relative to the true peak. In the example shown in Fig. 21, when the height of the true peak (the position of a nucleotide length X) is 1,000, the height of a +A peak (the position of nucleotide length X+1) is 500, and the height of a stutter peak located on the left of the true peak by one unit (the position of nucleotide length X-one unit length) is 600.

Using this basic waveform, peaks of practically observed waveform data shown in the middle row of Fig. 21 are judged. In the lower row of Fig. 21, the result of fitting the basic waveform to the practically observed data shown in the middle row is shown. This fitting is carried out by choosing the highest peak (Pmax) from the observed waveform data on the assumption that this highest peak corresponds to the true peak in the basic waveform, adjusting the entire height of the basic waveform such that the highest peak of the basic waveform becomes equal to the height of Pmax (no change in relative heights of individual peaks of the basic waveform), and laying this adjusted basic waveform on top of the observed waveform. In the lowest row of Fig. 21, the fitted waveform is indicated by white triangles, and the differences of peak height from the observed waveform are shown by vertical arrows.

There exist homozygote and heterozygote for a pair of microsatellites on a genome. Only one true peak emerges on the graph when an extracted DNA fragment is homozygotic, while two true peaks emerge on the graph when the extracted DNA fragment is heterozygotic. Therefore, it becomes necessary to fit and lay two waveforms on two true peak positions for the heterozygote. Hence, after fitting the basic waveform as described above, attention is given to a peak (Pmax') that shows the maximum difference in peak height between the fitted waveform and the observed waveform. To this Pmax' position, the basic waveform (peak height adjusted at the Pmax' position) is further fitted. When the result shows better fitting compared to that in the first fitting of the basic waveform, the extracted DNA fragment is judged to be a heterozygote, and when the result shows worse fitting compared to that in the first fitting of the basic waveform, the extracted DNA fragment is judged to be a homozygote.

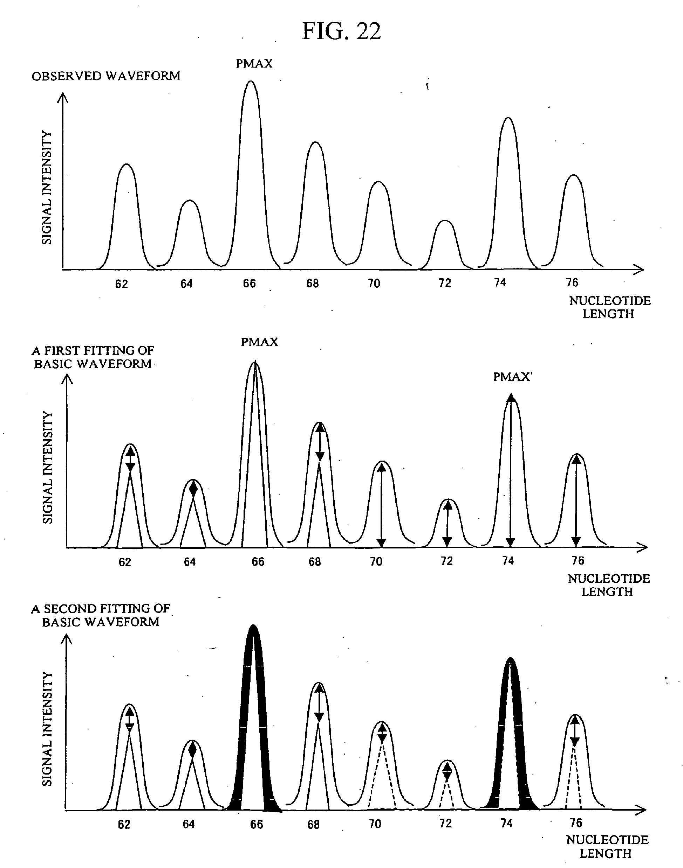

In the example shown in Fig. 21, fitting is better when thy waveform was fitted once, and thus the microsatellite contained in the DNA fragment extracted here is found to be a homozygote of 78 nucleotides. That is, the peak that appears at the position of 78 nucleotides is a true peak derived from the microsatellite of concern. On the other hand, in the example shown in Fig. 22, fitting is better when the waveform (the same as that in Fig. 21) was fitted twice, and thus the microsatellite contained in the DNA fragment extracted here is found to be a heterozygote of 66 and 74 nucleotides. That is, the peaks that appeared at the positions of 66 and 74 nucleotides are true peaks derived from the microsatellite of concern.

For example, Patent Documents 1 to 5, Non-patent Documents 1 to 5 employ this second method.
[Patent Document 1] U.S. Patent No. 5,541,067
[Patent Document 2] U.S. Patent No. 5,580,728
[Patent Document 3] U.S. Patent No. 5,876,933
[Patent Document 4] U.S. Patent No. 6,054,268
[Patent Document 5] U.S. Patent No. 6,274,317
[Non-patent Document 1] Perlin, M.W., et al., "Toward Fully Automated Genotyping: Allele Assignment, Pedigree Construction, Phase Determination, and Recombination Detection in Duchenne Muscular Dystrophy", Am. J. Hum. Genet. 55, 1994, p777-787
[Non-patent Document 2] Perlin, M.W., et al., "Toward Fully Automated Genotyping: Genotyping Microsatellite Markers by Deconvolution", Am. J. Hum. Genet. 57, 1995, p1199-1210
[Non-patent Document 3] Palsson, B., et al., "Using Quality Measures to Facilitate Allele Calling in High-Throughput Genotyping", Genome Research 9, 1999, p1002-1012
[Non-patent Document 4] Stoughton, R., et al., "Data-adaptive algorithms for calling alleles in repeat polymorphisms", Electrophoresis 18, 1997, p1-5
[Non-patent Document 5] Smith, J.R., et al., "Approach to Genotyping Errors Caused by Nontemplated Nucleotide Addition by Taq DNA Polymerase", Genome Research 5, 1995, p312-317
In L.T. Parker et al. "Peak height variations in automated sequencing of PCR products using TAQ dye-terminator Chemistry" Biotechniques, Informa Life Sciences Publishing, Westborough, MA, US, vol. 19, no. 1, 1995, pages 116-121, ISSN: 0736-6205, an allele calling program for determining allele calls from the fluorescence analysis result obtained from an electrophoresis experiment of PCR amplification products of a DNA marker is disclosed. The sequence information of the DNA fragment is used to determine the presence of noise peaks or artefacts in order to improve the accuracy of the allele-calling.

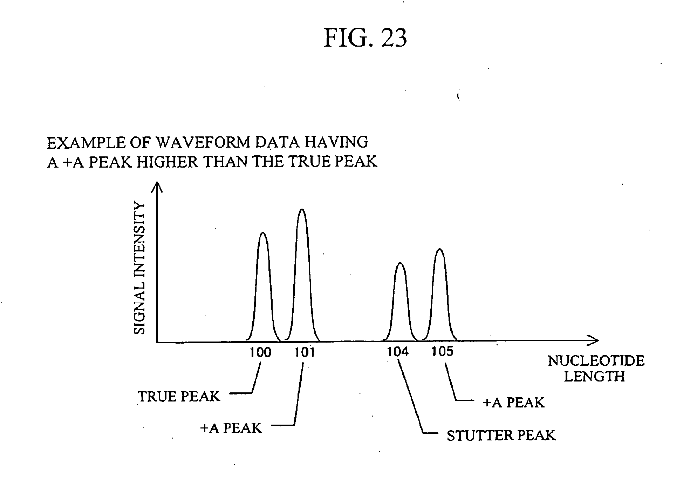

In the first method described above, however, when a +A peak higher than the true peak appears as shown in Fig. 23, peak judgment may fail because the highest peak is always judged to be a true peak. It should be noted that occurrence of this kind of phenomenon has been reported in Non-patent document 5.

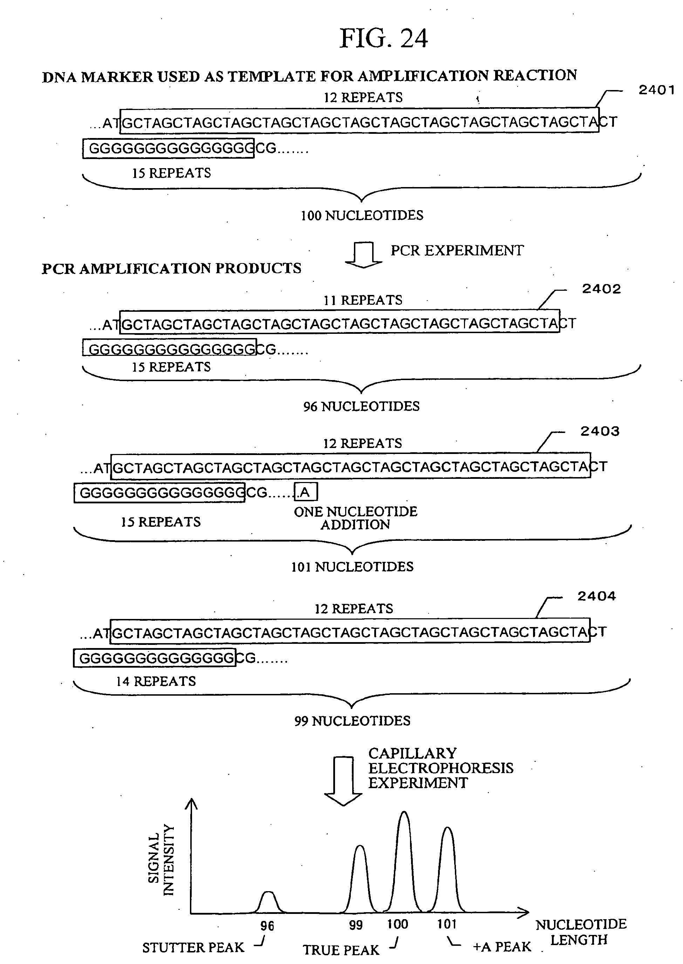

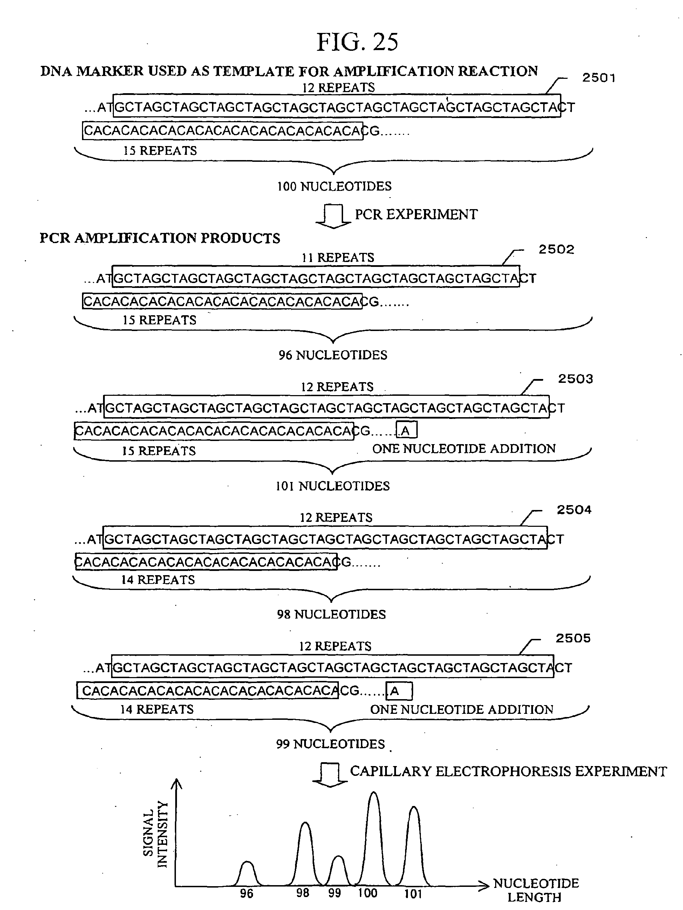

On the other hand, there is a problem in the second method that this technique cannot deal with noise peaks other than stutter peaks and +A peaks. An example in which the noise peaks other than stutter peaks and +A peaks appear in waveform data of the fluorescence analysis result obtained from an electrophoresis experiment of a DNA fragment is explained with reference to Figs. 24 and 25. In Fig. 24, a portion having repeats of a single nucleotide "G" is present in addition to a microsatellite portion consisting of repeats of "GCTA" unit in a DNA fragment 2401 used for a template of PCR amplification reaction. The "GCTA" unit is repeated twelve times, and "G" is repeated fifteen times, which forms a DNA fragment of 100 nucleotides as a whole. When this DNA fragment 2401 is amplified in a PCR experiment, products having an altered number of repeats of the microsatellite unit 2402, an additional "A" at the end 2403, and an altered number of repeats of the single nucleotide "G" 2404 are known to be generated as experimental noises. These DNA fragments 2402, 2403, and 2404 that have been amplified as noises are observed at the positions of 96, 101, and 99 nucleotide lengths in waveform data of an electrophoresis experiment as noises, respectively. The peaks appearing at the positions of 96 and 101 nucleotide lengths are a stutter peak and a +A peak, respectively, and the peak appearing at the position of 99 nucleotide length is a noise peak derived from the single nucleotide repeat portion not representing the microsatellite. On the other hand, in Fig. 25, a DNA fragment 2501 having a repeat portion of two nucleotides "CA" in addition to a microsatellite portion consisting of repeats of a "GCTA" unit is used as a template of a PCR reaction. As a result of PCR amplification of this template, products having not only an altered number of repeats of the microsatellite unit 2502 and an additional "A" at the end 2503 but also an altered number of repeats of the two nucleotide "CA" 2504, a further additional "A" at the end of the latter 2505, and the like are known to be generated as experimental noises. These DNA fragments 2502, 2503, 2504, and 2505 that were amplified as noises are observed at.the positions of 96, 101, 98, and 99 nucleotide lengths in waveform data of an electrophoresis experiment as noises, respectively.

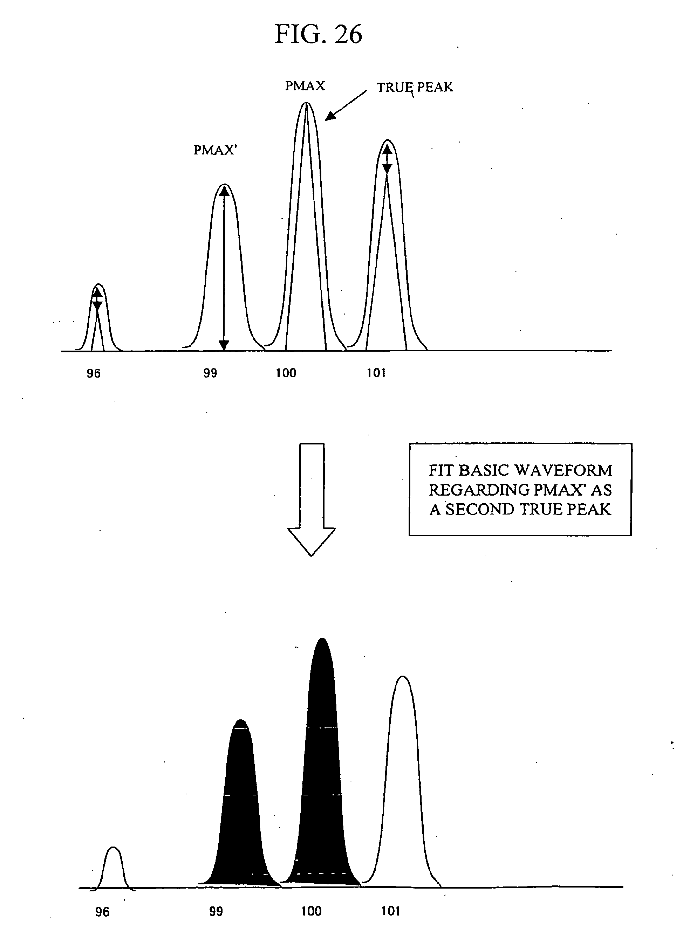

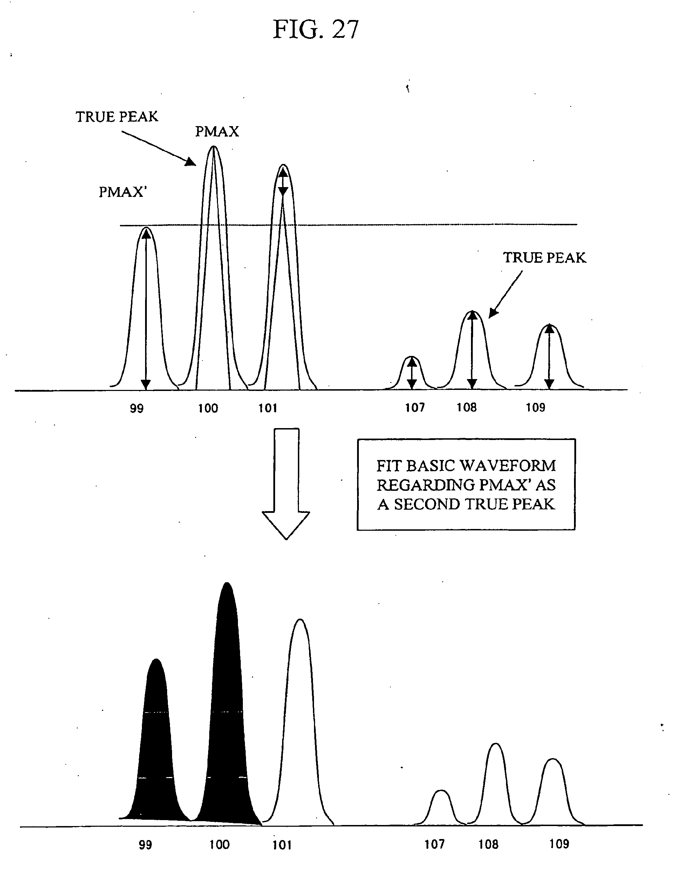

Since appearance of noise peaks other than stutter peaks and +A peaks is not assumed in conventional technology (the second method described above, etc.), there has been a problem that a correct peak judgment on the waveform data containing noise peaks as shown in Figs. 24 and 25 cannot be made. This point is explained with reference to Figs. 26 and 27. The waveform data shown in the upper row of Fig 26 is the same as that shown in Fig. 24, and a noise peak other than stutter peaks and +A peaks appears at the position of 99 nucleotides. When the second method described above is applied to this waveform data after the basic waveform is fitted to the maximum peak Pmax, the noise peak at the position of 99 nucleotides is selected as the peak Pmax' that shows a maximum difference in peak height between the fitted basic waveform and the observed waveform. As the result, the peak at the position of 100 nucleotides and the peak at the position of 99 nucleotides are misjudged to be true peaks. Fig. 27 illustrates the waveform data of a heterozygote. The true peaks appear at the positions of 100 and 108 nucleotides, and peaks other than stutter peaks and +A peaks appear at the positions of 99 and 107 nucleotides. When the second method described above is applied to this waveform data, a misjudgment that the noise peak at the position of 99 nucleotides is a true peak is made because the noise peak at the position of 99 nucleotides higher than the true peak at the position of 108 nucleotides is judged as Pmax'.

### SUMMARY OF THE INVENTION

The present invention was accomplished in light of the above-mentioned circumstances and provides a display method and a display apparatus that allows true peaks and noise peaks to be discriminated correctly and displayed in waveform data of a fluorescence analysis result obtained from an electrophoresis experiment of PCR amplification products of a DNA fragment. Particularly, the present invention provides the display method and the display apparatus that allow the true peaks to be discriminated correctly even when noise peaks other than conventionally well-known stutter peaks and +A peaks appear.

As a result of assiduous research in consideration of the above problem to be solved, the present inventors have devised a peak judging method having the following three features as a method of judging a correct peak for data of a waveform (hereinafter, referred to as "complex peak waveform") that contains noise peaks resulting from the presence of a repeat portion other than a microsatellite in a DNA fragment serving as a template in PCR amplification reaction in addition to conventionally well-known stutter peaks and +A peaks described above:
Feature 1; Whether a DNA marker is the one that generates a complex peak waveform is judged based on the sequence information of the DNA marker (the template in PCR amplification reaction), and a peak judging algorithm dedicated to complex peak waveform is applied to the DNA marker that generates a complex peak waveform.
Feature 2; Whether a DNA marker is the one that generates a complex peak waveform is judged by whether the number of repeats in a repeat portion, other than the microsatellite, that causes a complex peak waveform exceeds a predetermined threshold.
Feature 3; At the time of fitting the basic waveform for peak judgment of the complex peak waveform, the distance between a first fitting position and a second fitting position of the basic waveform is made longer than a unit length.

The above feature 1 is explained in detail. For example, a DNA marker having a sequence of "...ATGCTAGCTAGCTAGCTAGCTAGCTAGCTAGCTAGCTAGCTA GCTAGCTACTGGGGGGGGGGGGGGGCG..." that contains a microsatellite having a unit of four nucleotides "GCTA" is assumed. It is found from the sequence information that a sequence with repeats of one nucleotide "G" is contained in this DNA marker in addition to the microsatellite having the unit of "GCTA". In such a case, the DNA marker is judged to produce a complex peak waveform, and the peak judgment is carried out by applying the peak judging algorithm dedicated to complex peak waveform (peak judging algorithm employing the feature 3 below). A conventional peak judging method may be applied to the DNA marker that does not produce a complex peak waveform.

The feature 2 described above is explained in detail. When a repeat portion other than the microsatellite is contained in the DNA marker, a threshold is set in advance as to what number of repeats in that portion is at least necessary for producing a complex peak waveform. This threshold can vary depending on the kind of DNA marker, the experimental environment, the experimental protocol, and the like, and a user may set a value determined empirically (the present inventors set the threshold to about ten). This may also vary depending on the length of a repeat unit (nucleotide length) in the repeat portion. For this reason, it is desirable that a different threshold is allowed to be specified for each nucleotide length of the repeat unit. For example, the threshold is set to 12 for the repeats of one nucleotide unit, while the threshold is set to 10 for the repeats of two nucleotide unit, and so on.

The feature 3 described above is explained later in detail as an embodiment of the present invention.

Hence, according to the present invention, whether waveform data obtained from an electrophoresis experiment is the waveform data of a DNA marker that produces a complex peak waveform can be appropriately judged by the above features 1 and 2, and when the DNA marker is the one that produces a complex peak waveform, peak judgment can be made by the above feature 3 using the peak judging algorithm dedicated to complex peak waveform. For DNA markers other than that, plural kinds of methods for judging true peaks can be used properly to judge true peaks by existing methods. Further, the third feature allows true peaks in a waveform observed for a DNA marker that produces a complex peak waveform to be judged appropriately. In this way, judgment of true peaks can always be made not only for a DNA marker that produces a complex peak waveform but also for other DNA markers.

As a means to realize specifically the above features 1 to 3, the present invention provides a display apparatus according to claim 1, a display method according to claim 4 and a corresponding computer program. Preferable embodiments are defined in the dependent claims. In particular, the present invention provides a display apparatus to display results analyzed for the lengths of PCR amplification products of a DNA fragment containing a microsatellite. According to one aspect of the display apparatus of the present invention, the apparatus includes a complex peak waveform judging unit that judges whether or not noise peaks, other than stutter peaks with increased or decreased repeat units of the microsatellite in the DNA fragment corresponding to detection signals of the PCR amplification products and +A peaks with one adenine added to the DNA fragment corresponding to the detection signals of the PCR amplification products, are generated in the detection signals of the PCR amplification products based on sequence information on the DNA fragment; a peak discrimination processing unit that discriminates true peaks corresponding to the detection signals of the PCR amplification products of the DNA fragment by fitting a basic waveform, in which a pattern of appearance of stutter peaks and +A peaks in the detection signals of the PCR amplification products of the DNA fragment is made in a model for every kind of the DNA fragment, to the detection signals of the PCR amplification products; and a display processing unit that displays a discrimination result of true peaks by the peak discrimination processing unit, where the peak discrimination processing unit excludes peaks presumed to be noise peaks other than the stutter peaks and the +A peaks from fitting targets of the basic waveform when the complex peak waveform judging unit judges generation of the noise peaks other than the stutter peaks and the +A peaks in the detection signals of the PCR amplification products.

Herein, the complex peak waveform judging unit judges whether the noise peaks other than the stutter peaks and the +A peaks are generated in the detection signals of the PCR amplification products based on whether a repeat sequence with at least one nucleotide as a unit other than the microsatellite contained in the DNA fragment is present.

According to a preferred embodiment of the display apparatus of the present invention, the display apparatus is further provided with a user condition-setting unit in which a nucleotide length of the repeat unit and a threshold of the number of repeats with respect to the repeat sequence other than the microsatellite are set by a user as a condition of judgment in the complex peak waveform judging unit.

According to the display apparatus of the present invention, the peak discrimination processing unit excludes peaks presumed to be the noise peaks other than the stutter peaks and the +A peaks from the fitting targets of the basic waveform by making the distance between the first fitting position of the basic waveform and the second fitting position of the basic waveform separated more than the unit length of the microsatellite contained in the DNA fragment when the first fitting of the basic waveform to the detection signals of the PCR amplification products is further followed by the second fitting of the basic waveform to these signals.

According to a preferable embodiment of the display apparatus of the present invention, the display processing unit displays not only a graph of the detection signals of the PCR amplification products, sequence information of the DNA fragment, and a judgment result by the complex peak waveform judging unit but also the discrimination result of the true peaks by the peak discrimination processing unit.

The present invention provides a display method to display results analyzed for the lengths of PCR amplification products of a DNA fragment containing a microsatellite. According to the display method of the present invention, the method includes a complex peak waveform judging step to judge whether or not noise peaks, other than stutter peaks with increased or decreased repeat units of the microsatellite in the DNA fragment corresponding to detection signals of the PCR amplification products and +A peaks with one adenine added to the DNA fragment corresponding to the detection signals of the PCR amplification products, are generated in the detection signals of the PCR amplification products based on sequence information on the DNA fragment; a peak discrimination processing step to discriminate true peaks corresponding to the detection signals of the PCR amplification products of the DNA fragment by fitting a basic waveform, in which a pattern of appearance of stutter peaks and +A peaks in the detection signals of the PCR amplification products of the DNA fragment is made in a model for every kind of the DNA fragment, to the detection signals of the PCR amplification products; and a display processing step to display a discrimination result of true peaks in the peak discrimination processing step, where peaks presumed to be noise peaks other than the stutter peaks and the +A peaks are excluded from fitting targets of the basic waveform in the peak discrimination processing step when the noise peaks other than the stutter peaks and the +A peaks in the detection signals of the PCR amplification products are judged to be generated in the complex peak waveform judging step.

According to the display method of the present invention, whether the noise peaks other than the stutter peaks and the +A peaks are generated in the detection signals of the PCR amplification products is judged in the complex peak waveform judging step based on whether a repeat sequence having at least one nucleotide as a unit other than the microsatellite contained in the DNA fragment is present.

According to a preferred embodiment of the display method of the present invention, the display method is further provided with a user condition-setting step in which a nucleotide length of the repeat unit and a threshold of the number of repeats are set with respect to the repeat sequence other than the microsatellite by a user as a condition of judgment in the complex peak waveform judging step.

According to the display method of the present invention, peaks presumed to be the noise peaks other than the stutter peaks and the +A peaks are excluded from the fitting targets of the basic waveform in the peak discrimination processing step by making the distance between a first fitting position of a basic waveform and a second fitting position of a basic waveform separated more than the unit length of the microsatellite contained in the DNA fragment when the first fitting of the basic waveform to the detection signals of the PCR amplification products is further followed by the second fitting of the basic waveform to these signals.

According to a preferred embodiment of the display method of the present invention, not only a graph of the detection signals of the PCR amplification products, sequence information of the DNA fragment,
and a judgment result in the complex peak waveform judging step but also the discrimination result of the true peaks in the peak discrimination processing step is displayed in the display processing step.

The present invention also provides a program to execute any one of the display methods described above on the display apparatus.

As explained in the foregoing, according to the display method and the display apparatus of gene information of the present invention, the waveform data of a fluorescence analysis result that is obtained from an electrophoresis experiment of PCR amplification products of a DNA fragment is judged as to whether the waveform is the one (complex peak waveform) containing noise peaks other than stutter peaks and +A peaks based on the sequence information of the DNA fragment, and true peaks can be judged based on the judgment result using an appropriate peak judging algorithm. Since a criterion to judge whether the waveform is a complex peak waveform can be arbitrarily set by a user, accuracy of judgment processing for true peaks can be improved to a significant degree by setting an optimal condition of judgment for every target DNA marker for analysis.

### BRIEF DESCRIPTION OF THE DRAWINGS

Fig. 1 is a schematic functional block diagram showing an internal composition of a display system of gene information constructed as an embodiment of the present invention;
Fig. 2 is a diagram showing a data structure of waveform data contained in a data memory of the display system of gene information shown in Fig. 1;
Fig. 3 is a diagram showing another data structure of the waveform data contained in the data memory of the display system of gene information shown in Fig. 1;
Fig. 4 is a diagram showing still another data structure of the waveform data contained in the data memory of the display system of gene information shown in Fig. 1;
Fig. 5 is a diagram showing a data structure of sequence data contained in the data memory of the display system of gene information shown in Fig. 1;
Fig. 6 is a flow chart showing a flow of a whole process for peak judgment of waveform data of a DNA marker in the display system of gene information shown in Fig. 1;
Fig. 7 represents a screen of a user interface displayed on a display apparatus to prompt the user to input a condition concerning a repeat sequence;
Fig. 8 represents an example of a screen displaying a graph of the waveform data and a result of peak judgment as a result of a process for peak judgment;
Fig. 9 is a flow chart showing in detail a process for judging complex peak waveform generation (step 603) in the flow chart shown in Fig. 6;
Figs. 10A and 10B represent a specific mode of a masking process of a microsatellite portion in the sequence of the DNA marker (step 901) in the flow chart shown in Fig. 9, where Fig. 10A illustrates an example of masking, and Fig 10B illustrates another example of masking;
Fig. 11 is a flow chart showing in detail the process for peak judgment of the complex peak waveform (the step 604) in the flow chart shown in Fig. 6;
Fig. 12 illustrates an example of a practical analysis procedure for the waveform data of the DNA marker according to the flow chart shown in Fig.11;
Fig. 13 illustrates another example of the practical analysis procedure for the waveform data of the DNA marker according to the flow chart shown in Fig. 11;
Fig. 14 illustrates still another example of the practical analysis procedure for the waveform data of the DNA marker according to the flow chart shown in Fig.11;
Fig. 15 represents an example of the display screen displaying a peak judgment result obtained when an analysis for the waveform data of the DNA marker was carried out according to the procedure shown in Fig. 12;
Fig. 16 represents another example of the display screen displaying a peak judgment result obtained when an analysis for the waveform data of the DNA marker was carried out according to the procedure shown in Fig. 13;
Fig. 17 represents still another example of the display screen displaying a peak judgment result obtained when an analysis for the waveform data of the DNA marker was carried out according to the procedure shown in Fig. 14;
Fig. 18 is an illustration to explain a microsatellite appearing on a genome;
Fig. 19 is a schematic illustration of an experimental procedure to extract and amplify a DNA fragment of a microsatellite portion by PCR and gel electrophoresis;
Fig. 20 is an illustration to explain stutter peaks and +A peaks representing noises that occur during the course of PCR and electrophoresis experiments;
Fig. 21 is an illustration to explain a conventional technology by which noise peaks in the waveform data of fluorescence analysis result obtained from an electrophoresis experiment of a DNA fragment are discriminated using a waveform in which the way noise peaks appear is made in a model;
Fig. 22 is another illustration to explain the conventional technology by which noise peaks in the waveform data of a fluorescence analysis result obtained from the electrophoresis experiment of a DNA fragment are discriminated using the waveform in which the way noise peaks appear is made in a model;
Fig. 23 illustrates an example in which a +A peak higher than a true peak appears in the waveform data of a fluorescence analysis result obtained from the electrophoresis experiment of a DNA fragment;
Fig. 24 illustrates an example in which noise peaks other than stutter peaks and + A peaks appear in the waveform data of a fluorescence analysis result obtained from the electrophoresis experiment of a DNA fragment;
Fig. 25 illustrates another example in which noise peaks other than the stutter peaks and the +A peaks appear in the waveform data of a fluorescence analysis result obtained from the electrophoresis experiment of a DNA fragment;
Fig. 26 is an illustration to explain a problem of a peak judging method according to the conventional technology; and
Fig. 27 is an illustration to explain another problem of the peak judging method according to the conventional technology.

### DETAILED DESCRIPTION OF THE PREFERRED EMBODIMENTS

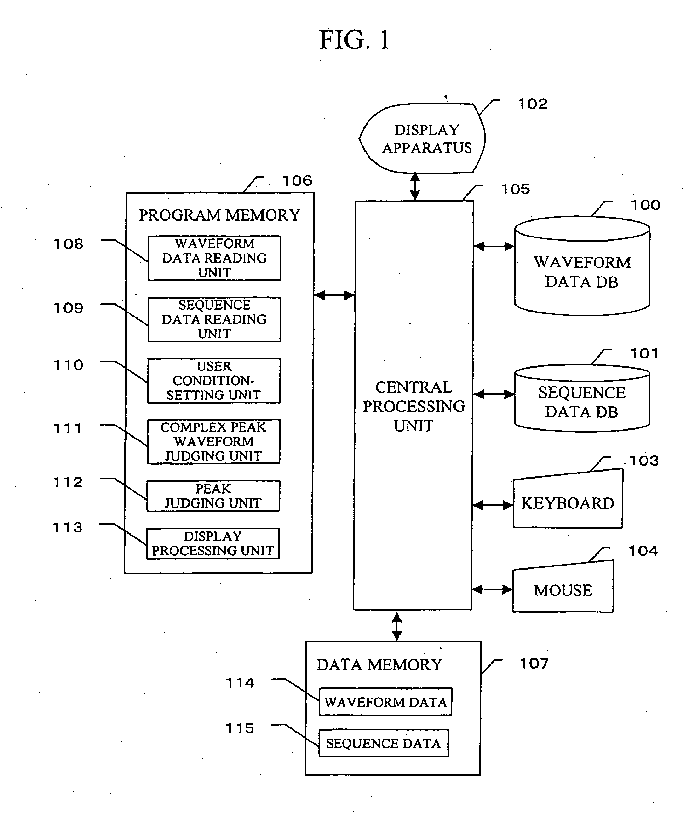

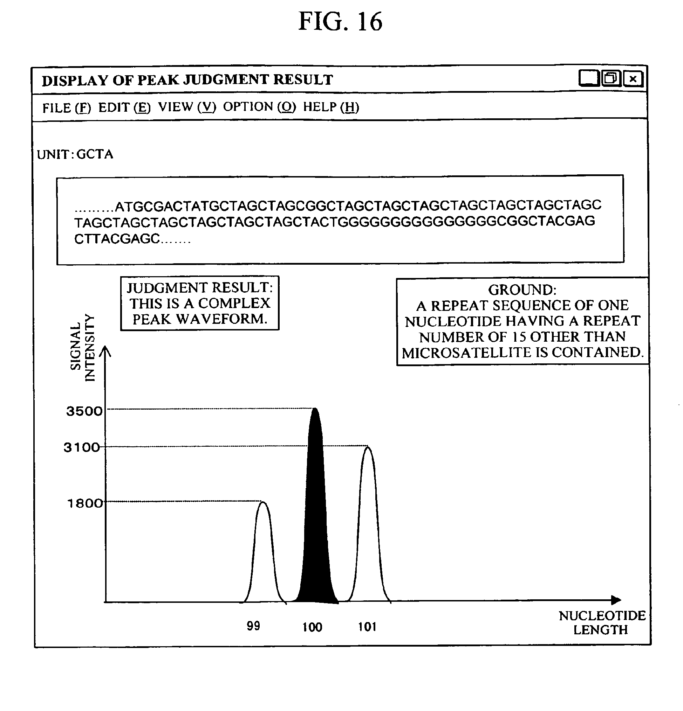

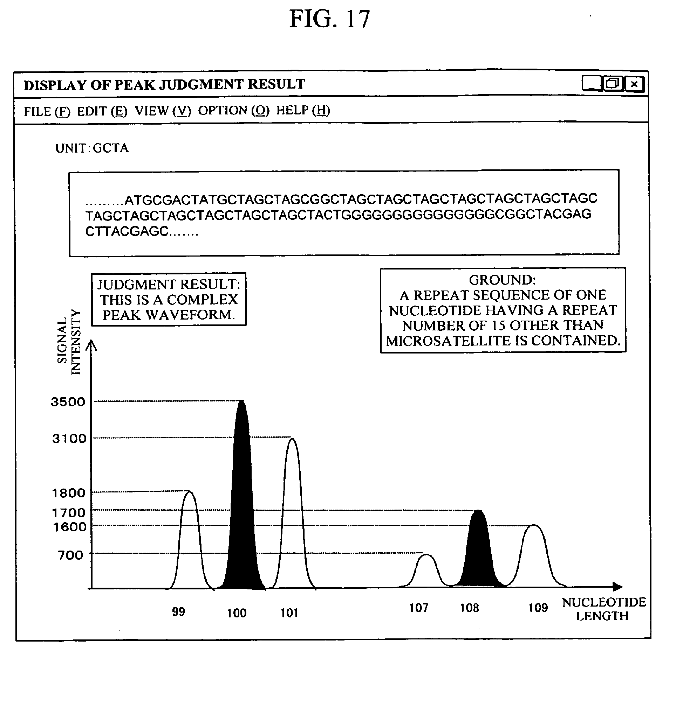

Hereinafter, best mode for carrying out the display method and the display apparatus of gene information of the present invention is explained in detail with reference to the accompanying drawings. Figs. 1 to 17 are illustrations to exemplify embodiments of the present invention. In these illustrations, the portions designated by the same reference numerals indicate the same, and their fundamental compositions and operations are the same.

Fig. 1 is a schematic functional block diagram showing an internal composition of a display system of gene information constructed as an embodiment of the present invention. This display system of gene information is provided with a waveform data DB 100 that stores waveforms, for every DNA marker, obtained by fluorescence analysis of PCR amplification products after PCR and electrophoresis experiments, a sequence DB 101 that stores sequence information of each marker DNA, a display apparatus 102 to display the waveform data and its analysis result in a graph, a keyboard 103 and a pointing device 104 such as mouse that are used for operations to select an individual or a peak on a displayed graph and the like, a central processing unit 105 that executes necessary computation processing, control processing, and the like, a program memory 106 that stores programs necessary for processing by the central processing unit 105, and a data memory 107 that stores data necessary for processing by the central processing unit 105.

The program memory 106 includes a waveform reading unit 108 that reads waveform data of a DNA marker to be targeted for peak judgment from the data memory 107, a sequence data reading unit 109 that reads DNA sequence information on the DNA marker whose waveform has been read from the data memory 107, a user condition-setting unit 110 that allows a user to designate a condition serving as a criterion for judging whether the DNA marker is the one that generates a complex peak waveform from the sequence information of the DNA marker to be targeted for peak judgment, a complex peak waveform judging unit 111 that judges whether the DNA marker is the one that generates a complex peak waveform by referring to the sequence data of the DNA marker to be targeted for peak judgment according to the condition designated by the user, a peak judging unit 1 12 that processes peak judgment of the waveform data of the DNA marker in accordance with the result of judgment of the complex peak waveform, and a display processing unit 113 that displays the result of peak judgment.

The data memory 107 includes waveform data 114 that stores waveform data of plural individuals for each DNA marker and sequence data 115 of each marker. The waveform data 114 and the sequence data 115 are stored in the data memory 107 by reading from the wave form DB 100 and the sequence data DB 101.

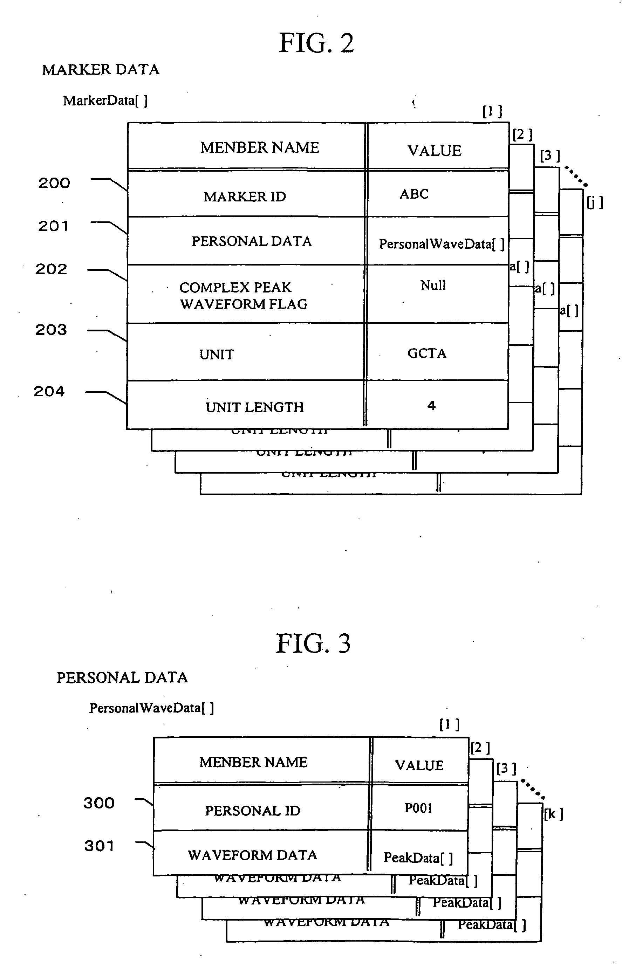

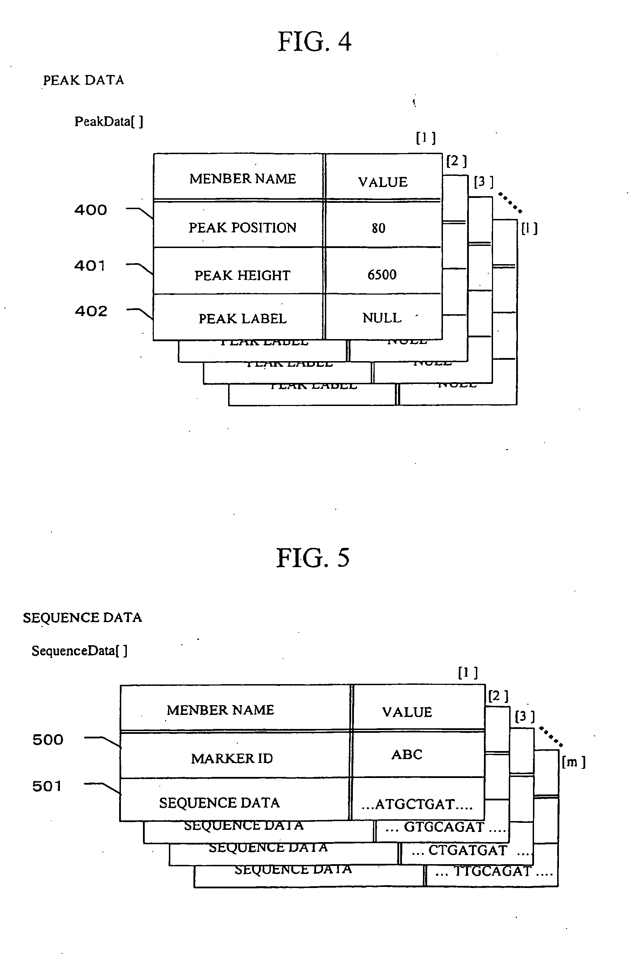

Figs. 2 to 4 represent data structures of the waveform data 114 included in the data memory 107. The data structure, MarkerData[], shown in Fig. 2 includes a marker ID 200 to identify each DNA marker with respect to j pieces of DNA markers, a pointer 201 to PersonalWaveData[] that indexes waveform data appearing for each individual, a complex waveform flag 202 that shows whether a complex peak wave form appears or not, and data showing a unit 203 contained in a microsatellite and a unit length 204 thereof. Here, data of the complex waveform flag 202 possesses a null value when computation has not yet been performed. The data structure, PersonalWaveData[], shown in Fig. 3 contains data showing a personal ID 300 to identify each individual with respect to k pieces of individuals and a pointer to each personal waveform data, PeakData[]. The data structure, PeakData[], shown in Fig. 4 contains data showing a peak position (nucleotide length) 400 at which each peak appears, a peak height 401 for each peak, and a peak label 402 that represents whether a peak is a true peak or a noise peak (stutter peak, +A peak, or other peak). Here, data of the peak label 402 possesses a null value when analysis has not yet been performed.

Fig.5 represents a data structure of the sequence data 115 included in the data memory 107. A data structure, SequenceData[], shown in Fig. 5 contains data showing a marker ID 500 to identify each DNA marker with respect to m pieces of DNA markers and data showing nucleotide sequence 501 of each DNA marker.

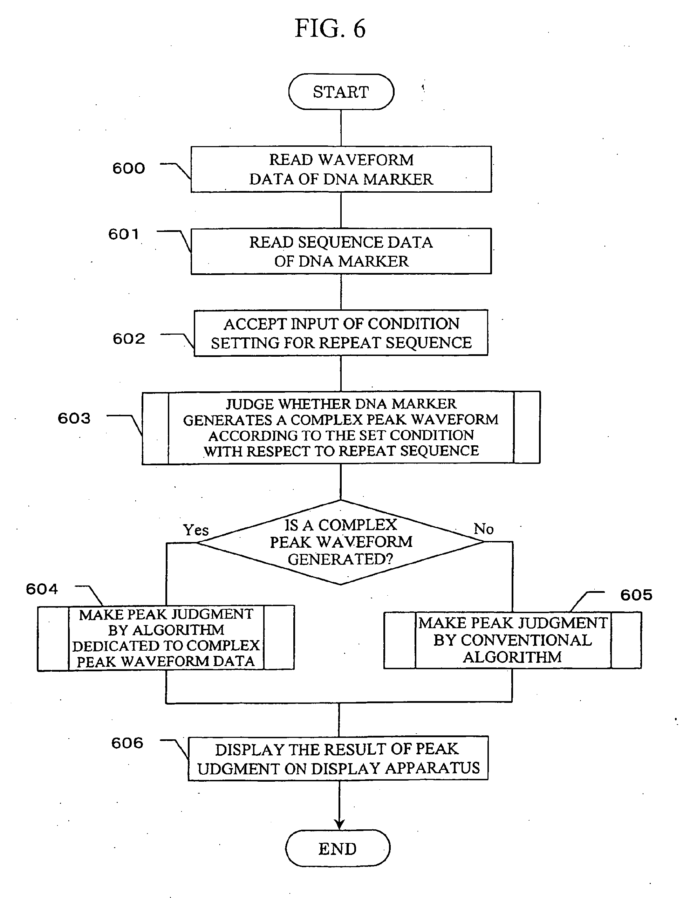

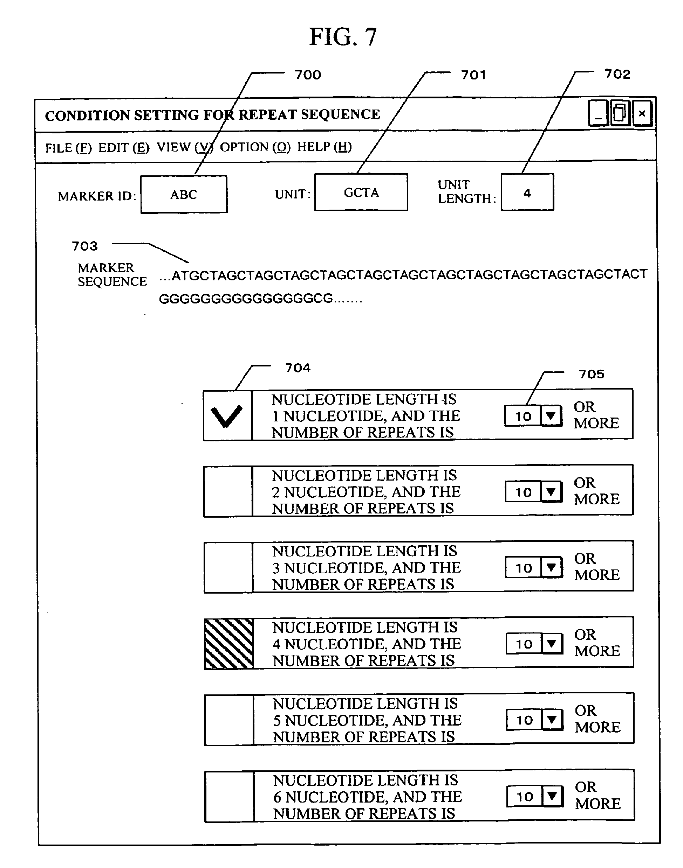

Fig. 6 is a flow chart showing a flow of a whole process for peak judgment of the waveform data of a DNA marker in the display system of gene information of the present embodiment. Each step explained below is executed by each of the processing units 108 to 113 in the program memory 106. In Fig. 6, first, the waveform reading unit 108 retrieves waveform data of a DNA marker to be processed for peak judgment from the data memory 107 (step 600). The data corresponding to one of the data structure MarkerData[] shown in Fig. 2 is read. The sequence data reading unit 109 retrieves the sequence data of the DNA marker read in the step 600 from the data memory 107 (step 601). The data corresponding to one of the data structure SequenceData[] shown in Fig. 5 is read. The user condition-setting unit 110 allows the user to designate a condition serving as a criterion of judging whether the DNA marker generates a complex peak waveform from the sequence information of the DNA marker to be targeted for peak judgment (step 602). Specifically, a screen for user interface as shown in Fig. 7 is displayed on the display apparatus 102, thereby prompting the user to perform an input operation with the keyboard 103 and the mouse 104.

In the screen of Fig. 7, a marker ID 700 of the DNA marker whose waveform has been read in the step 600, a unit 701 of a microsatellite that is present in the DNA marker, a unit length 702 of the DNA marker, a sequence 703 of the DNA marker, and checkboxes 704 and pull-down menus 705 provided for the user to designate a condition are displayed. The display items 701 to 703 are retrieved from the waveform data structure MarkerData[] and the sequence data structure SequenceData[]. Since the user can understand that extra sequence repeats of a single nucleotide "G" are contained in addition to the microsatellite by looking at the sequence 703 of the DNA marker on this screen, the user can check the uppermost checkbox. When the checkbox is checked by the user, the pull-down menu for the item becomes effective, and the user can set the number of repeats for the repeat sequence that becomes a threshold to judge generation of a complex peak waveform. This number of repeats is set to the default of 10. A repeat sequence having the same nucleotide length as the unit length of the microsatellite contained in the DNA marker is designed so as not to be selectable (only the item corresponding to four nucleotides is shown in gray color in the illustration). This is because the noise peaks arising from such a repeat sequence appear at the same positions as those where stutter peaks for a true peak appear, thereby making it unnecessary to judge peaks by discriminating these noise peaks from the stutter peaks. For additional information for making a judgment at the time of the condition setting, information on the primer sequence, allele frequency, and the like may be displayed besides displaying information on the sequence of the DNA marker and the microsatellite unit. The foregoing is the explanation relating to the step 602 in the flow chart shown in Fig. 6.

Subsequently, the complex peak waveform judging unit 111 judges whether a complex peak waveform appears from the sequence data of the DNA marker read in the step 601 according to the condition concerning the repeat sequence other than the microsatellite that has been set in the step 602 (step 603). The processing of this judgment is explained later in detail.

In the step 603, when a judgment that a complex peak waveform is generated is made, the peak judging unit 1 12 performs peak judgment of the waveform data with the use of a peak judging algorithm dedicated to complex peak waveform (described later in detail) (step 604). When a judgment that a complex peak waveform is not generated is made in the step 603, the peak judging unit 112 performs peak judgment of the waveform data with the use of a conventional peak judging algorithm (step 605). Here, the conventional peak judging algorithm performs peak judgment automatically on a computer based on peak judging methods disclosed in Patent documents 1 to 5 and Non-patent documents 1 to 4. It should be noted that whichever algorithm may be used, each peak appearing in the waveform data of the DNA marker read in the step 600 is judged to be any one of a true peak, +A peak, and stutter peak. This result of peak judgment is written in the peak label 402 of the data structure PeakData[] shown in Fig. 4.

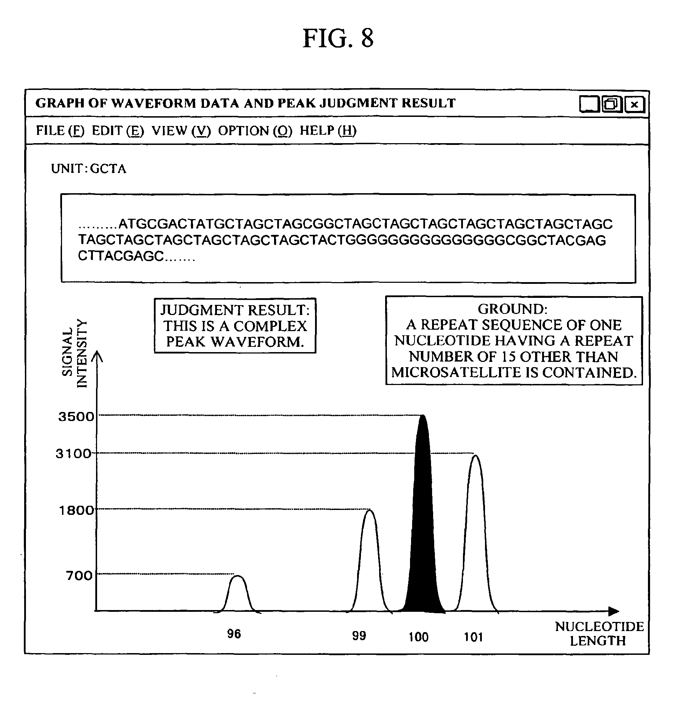

Then, the display processing unit 113 displays a graph of the waveform data and the result of peak judgment for each individual on the display apparatus 102 (step 606). Fig. 8 illustrates an example of display screen for the graph of the waveform data and the result of peak judgment. On the screen shown in Fig. 8, the unit and the sequence of the DNA marker, the waveform data, and the result of the peak judgment and the ground for the judgment on the waveform data are displayed. As to the result of the peak judgment, a method to display a peak judged to be a true peak (peak of 100 nucleotides in the illustration) in the waveform data in a color is employed, and the result that this waveform data is a complex peak waveform data and its ground are displayed. Since the peak judging unit 112 discriminates among a true peak, +A peaks, and stutter peaks as described above, the respective peaks may be displayed so as to be identified.

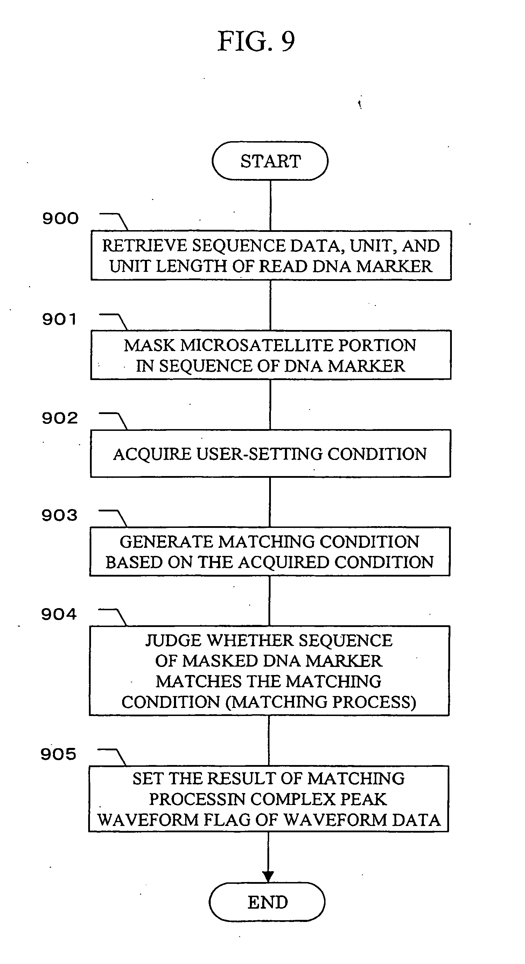

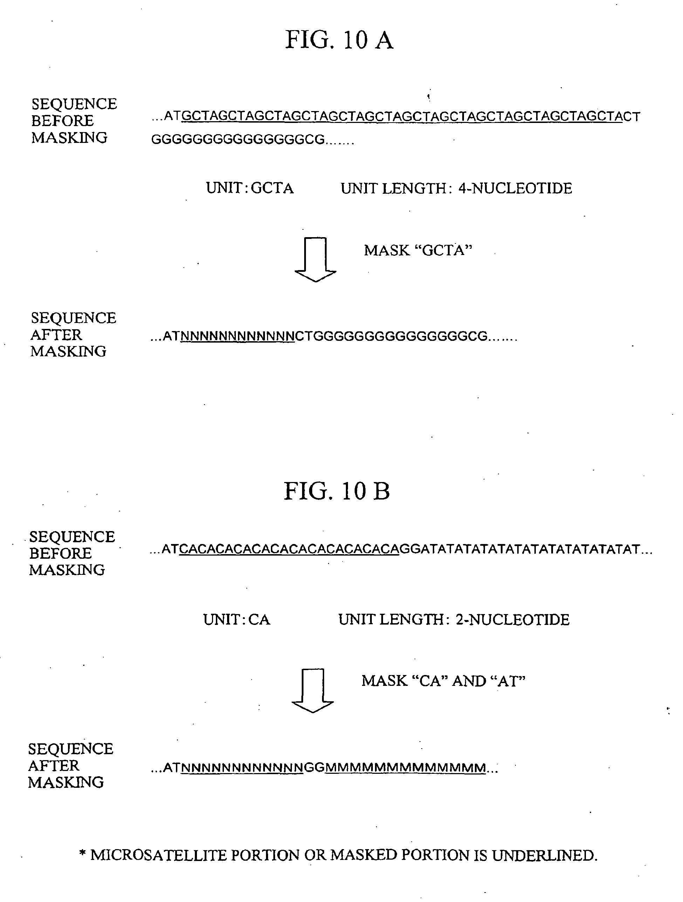

Fig. 9 is a flow chart showing a process for judging a complex peak waveform generation (the step 603) in the flow chart shown in Fig. 6 in detail. In Fig.9, first, the unit and the unit length of the microsatellite and the sequence of the DNA marker are retrieved from the waveform data (the data structure MarkerData[]) and the sequence data (the data structure SequenceData[]) of the DNA marker read in the steps 600 and 601 (step 900). The microsatellite portion in the sequence of the retrieved DNA marker is masked (step 901). Specific modes of this mask processing are shown in Fig. 10. In an example shown in Fig. 10A, the unit is "GCTA" and the unit length is four nucleotides; therefore a process to replace "GCTA" in the sequence with "N" is conducted. In another example shown in Fig. 10B, the unit is "CA and "the unit length" is two nucleotides. In such a case, the unit "CA" that is a unit of the microsatellite and the unit "AT" that is a unit of repeat sequence other than the microsatellite are both masked. In this way, it is judged in a later process that no repeat sequence other than the microsatellite is contained in the sequence of this DNA marker. This is because noise peaks arising from changes in the number of "AT" repeats and noise peaks arising from changes in the number of repeats in the microsatellite portion (stutter peaks) appear at the positions of the same nucleotide lengths, therefore making it unnecessary to judge as being complex peak waveform data.

Then, the condition set by the user in the step 602 in Fig. 6 is acquired (step 902). Specifically, the content designated by the user in the checkbox 704 and the pull-down menu 705 on the screen shown in Fig. 7, that is, the threshold for the number of repeats set for each nucleotide length as to the repeat sequence other than the microsatellite is acquired. From the acquired user setting condition, a matching condition to apply to the sequence of the DNA marker is generated (step 903). For example, when a condition that the nucleotide length is one and the threshold for the number of repeats is 10 is set, a matching condition that the repeats of A is 10 times or more, the repeats of T is 10 times or more, the repeats of G is 10 times or more, and the repeats of C is 10 times or more, is generated. When a condition that the nucleotide length is two and the threshold for the number of repeats is 10 is set, a matching condition that the number of repeats of any one of AT, AC, AG, TA, TC, TG, CA, CT, CG, GA, GT, and GC is 10 or more is set.

Whether the sequence of the DNA marker processed for the masking in the step 901 matches the matching condition generated in the step 903 is judged (step 904). For example, when the matching condition is "10 or more repeats of any one of A, T, G, and C", the sequence "...ATNNNNNNNNNNNNCTGGGGGGGGGGGGGGGCG....." after masking shown in Fig. 10A matches this matching condition. To match a matching condition means that the DNA marker is the one that generates a complex peak waveform. The result of this matching process is stored in the complex peak waveform flag in the data structure MarkerData[] of the waveform data (step 905). When the sequence of the DNA marker matches the matching condition, the complex peak waveform flag is indicated as true. When it does not match the matching condition, the complex peak waveform flag is indicated as false.

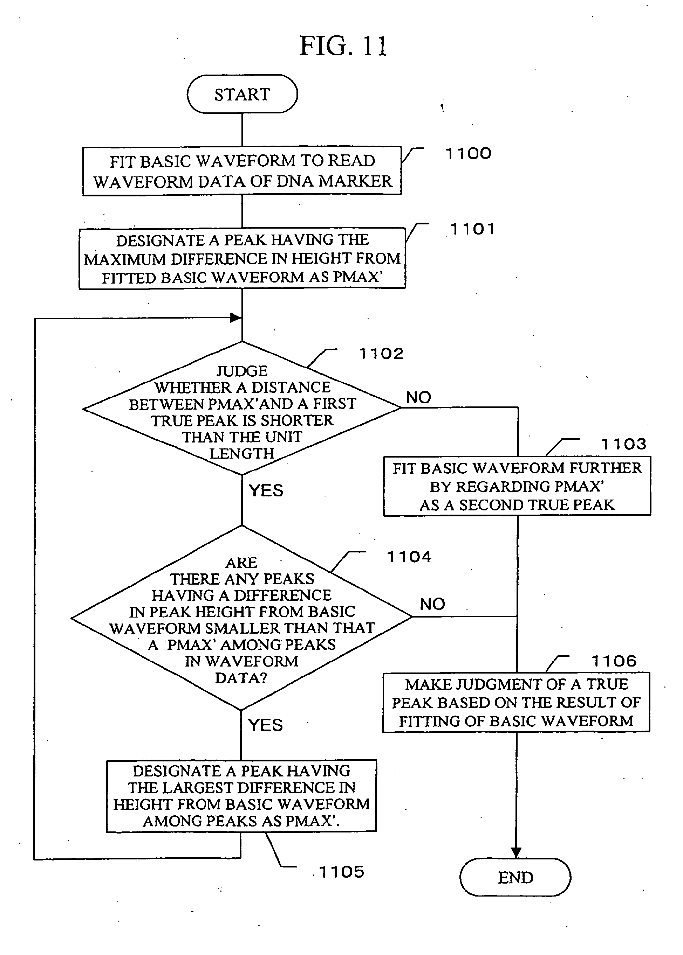

Fig. 11 is a flow chart showing in detail a process for peak judgment (the step 604) of a complex peak waveform in the flow chart shown in Fig. 6. In Fig. 11, first, a basic waveform set in advance is fitted to the waveform data of the DNA marker acquired in the step 600 (step 1100). A peak having a maximum difference in peak height from the fitted basic waveform is designated as Pmax' (step 1101). Then, whether the distance between the position of the selected Pmax' and the position of the peak assigned as a first true peak at the time of fitting the basic waveform is shorter than the unit length of the microsatellite is judged (step 1102). For example, When the unit length is four nucleotides and the position fitted to the true peak by fitting the basic waveform is 100 nucleotides, the subsequent process branches dependent on whether the selected position of Pmax' lies between 97 and 103 nucleotides. When the distance between the first true peak and Pmax' is equal to or longer than the unit length, the basic waveform is fitted further by regarding the position of Pmax' as a second true peak (step 1103).

In the step 1102, when the distance between the first true peak and Pmax' is shorter than the unit length, the differences in height between each peak in the waveform data and the fitted basic waveform are computed for the entire peaks, and whether there is any peak having a value smaller than the difference in peak height at Pmax' is judged (step 1104). When there is no such peak, the process is advanced to judgment of a true peak without performing a second fitting of the basic waveform. When there are peaks having smaller differences in peak height from the basic waveform compared with that at Pmax', a peak having the largest difference in the height is chosen, and this peak is redefined as Pmax', then returning to the step 1102 (step 1105). After having fitted the basic waveform once or twice in this way in the steps 1102 to 1105, a true peak is determined based on these results (step 1106). In analogy with conventional technology for peak judgment, when the second fitting of the basic waveform was performed in the step of 1103, the first fitting and the second fitting are compared, and the better fitting result of the two is employed (either homozygote or heterozygote is determined). The processes in the steps 1100, 1101, 1103, and 1106 are carried out in a manner similar to those in conventional technology described above.

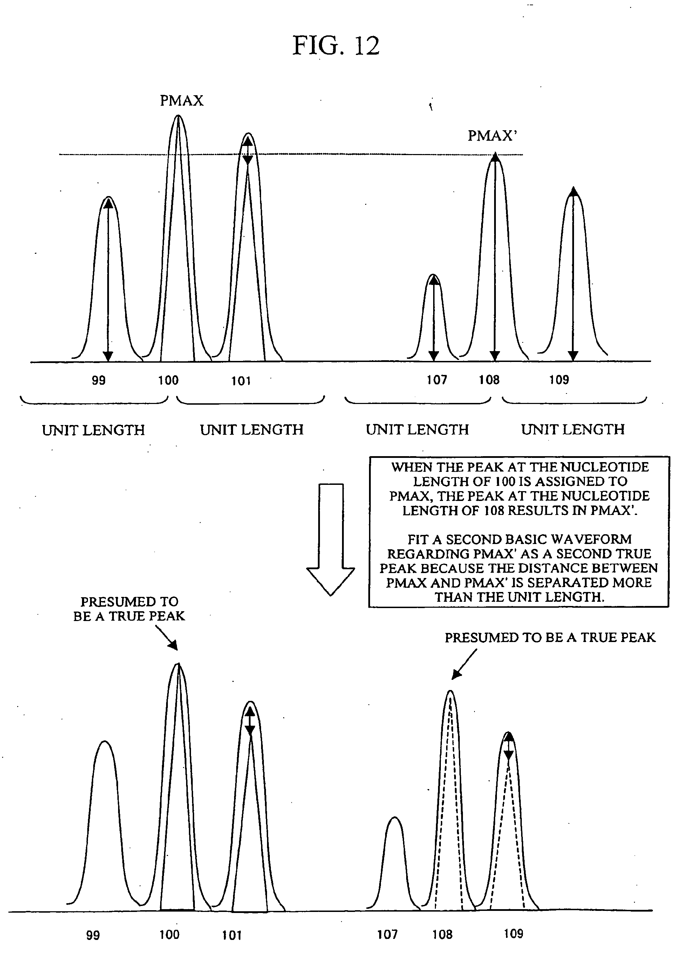

Figs. 12 to 14 represent practical procedures for analyzing the waveform data of the DNA marker according to the flow chart shown in Fig. 11. Here, the unit length of the microsatellite is four nucleotides, and the true peak is represented by the highest peak in the basic waveform. In Fig. 12, when the basic waveform is fitted by assigning the position of the nucleotide length of 100 as Pmax, the position of the nucleotide length of 108 results in Pmax'. Since the distance between the peak presumed to be a first true peak (i.e. Pmax) and Pmax' is longer than the unit length, the step is advanced from the step 1102 in Fig. 11 to the step 1103, and the second fitting of the basic waveform is performed to Pmax'. From the state of the second fitting of the waveform, the peak at the nucleotide length of 100 and the peak at the nucleotide length of 108 are judged to be true peaks, respectively, in this waveform.

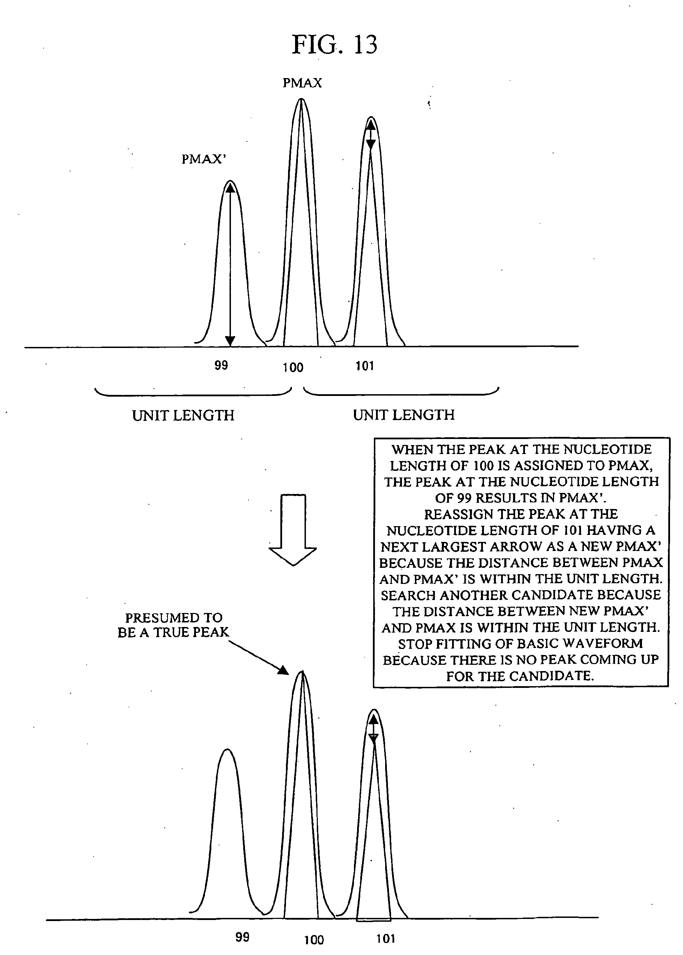

In Fig. 13, when the basic waveform is fitted by assigning the position of the nucleotide length of 100 as Pmax, the position of the nucleotide length of 99 results in Pmax'. Since the distance between the peak presumed to be a first true peak (i.e. Pmax) and Pmax' lies within the unit length, the step is advanced from the step 1102 in Fig. 11 to the steps 1104 and 1105, and the peak at the position of the nucleotide length of 101 that has the second largest difference in peak height from the basic waveform is reassigned as a new Pmax'. However, the distance between this new Pmax' and the Pmax lies also within the unit length, and therefore the step is advanced from the step 1102 to the step 1104 to search for another peak. Since there exists no peak having a difference in peak height smaller than that of Pmax', the process is advanced to peak judgment only with the first fitting of the basic waveform.

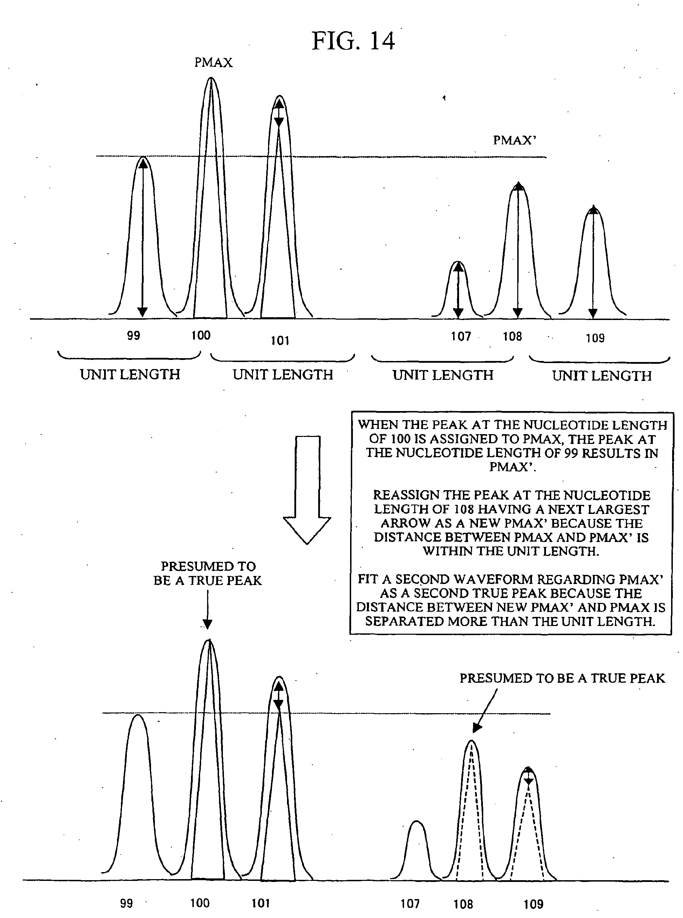

In Fig. 14, when the basic waveform is fitted by assigning the position of the nucleotide length of 100 as Pmax, the position of the nucleotide length of 99 results in Pmax': Since the distance between the peak presumed to be a first true peak (i.e. Pmax) and the Pmax' lies within the unit length, the step is advanced from the step 1102 in Fig. 11 to the steps 1 1 04 and 1105, and the peak at the position of nucleotide length of 108 having the next largest difference in peak height from the basic waveform is reassigned as a new Pmax'. Since the distance between this new Pmax' and Pmax is larger than the unit length, the step is advanced from the step 1102 to the step 1103, and a second fitting of the waveform is performed. From the state of the fitting of the waveform performed twice, the peak at the nucleotide length of 100 and the peak at the nucleotide length of 108 are judged to be true peaks, respectively, in this waveform data.

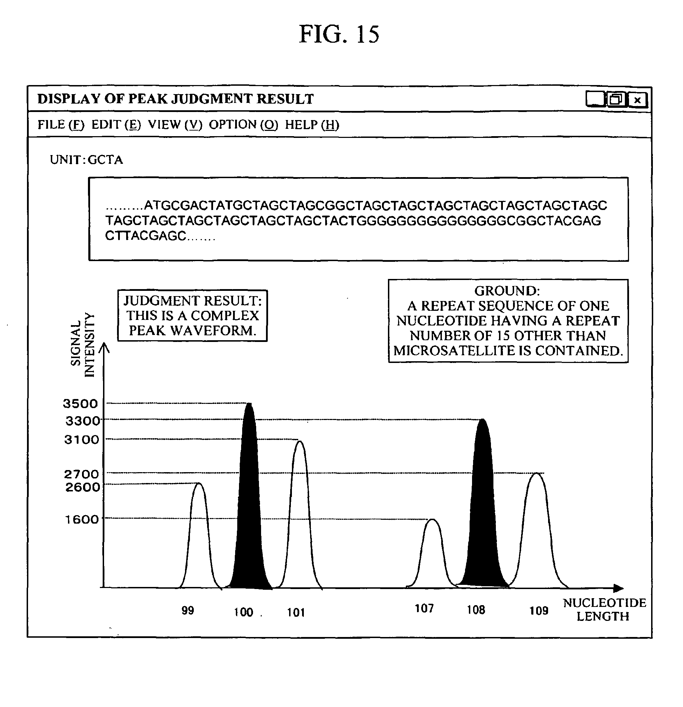

Examples of display screen showing the results of peak judgment that was made for analysis of the waveform data of the DNA marker according to the procedures shown in Figs. 12 to 14 are shown in Figs. 15 to 17, respectively. Displaying both the waveform data of the DNA marker and the sequence allows the user to receive the result of peak judgment as well as to confirm the sequence resulting in noise peaks with ease. This is also useful for the user to obtain information on the relation between DNA marker sequence and its emerging waveform.

When the results of peak judgment according to the gene information display system of the present embodiments shown in Figs. 12 to 17 and the results of peak judgment by conventional peak judging methods shown in Fig. 26 and 27 are compared, it is understood that the problem associated with the conventional technology that peaks are misjudged by appearance of noise peaks other than stutter peaks and +A peaks is eliminated in the gene information display system of the present embodiments. It is also understood that true peaks can be correctly identified irrespective of whether a DNA marker is homozygote or heterozygote. It should be noted that the basic waveform in which the true peak is higher than other noise peaks is used in Fig. 12 to 17. However, when a DNA marker in which a +A peak higher than the true peak appears is targeted for analysis, a basic waveform of that kind may be used. In this way, peak judgment can be correctly made irrespective of whether the highest peak is the true peak or a noise peak.

In the foregoing, the display method and the display apparatus of gene information of the present invention have been explained by showing the specific embodiments. However, the present invention is not limited to these embodiments.

The display method and the display apparatus of gene information of the present invention can be applied not only to individual genotyping technology with the aim of searching for genes affecting phenotypes such as diseases but also to individual genotyping technology with the aim of searching for genes affecting phenotypes other than diseases, individual genotyping technology in DNA identification, and the like. Further, genes of not only human but also agricultural products and marine products can be targeted.

In the above explanation, although electrophoresis was referred for examining a marker DNA fragment amplified by PCR, the present invention can also be applied to experimental techniques other than that. For example, noise peaks can also be properly processed in the analysis of waveform data obtained by matrix assisted laser desorption ionization time of flight mass spectrometry (MALDI-TOF-MS), in which PCR amplification products are ionized by a laser irradiation and their masses are determined, by using the display method and the display apparatus of gene information of the present invention.

The display method and the display apparatus of gene information of the present invention can be utilized by being mounted, for example, on a personal computer used as an experimental data analysis apparatus.

## Claims

1. A display apparatus for discriminating true peaks from noise peaks in waveform data of a fluorescence analysis result obtained from an electrophoresis experiment of PCR amplification products of a DNA fragment containing a microsatellite and displaying the results, the display apparatus comprising:
a complex peak waveform judging unit that judges whether or not noise peaks, other than stutter peaks with increased or decreased repeat units of the microsatellite in the DNA fragment corresponding to detection signals of the PCR amplification products and +A peaks with one adenine added to the DNA fragment corresponding to the detection signals of the PCR amplification products are generated in the detection signals of the PCR amplification products based on sequence information of the DNA fragment;
a peak discrimination processing unit that discriminates true peaks corresponding to the detection signals of the PCR amplification products of the DNA fragment by fitting a basic waveform, in which a pattern of appearance of stutter peaks and +A peaks in the detection signals of the PCR amplification products of the DNA fragment is made in a respective model for every kind of the DNA fragment, to the detection signals of the PCR amplification products;
a display processing unit that displays a discrimination result of true peaks by the peak discrimination processing unit,
wherein the peak discrimination processing unit excludes peaks presumed to be noise peaks other than the stutter peaks and the +A peaks from fitting targets of the basic waveform when the complex peak waveform judging unit judges generation of the noise peaks other than the stutter peaks and the +A peaks in the detection signals of the PCR amplification products,
wherein the complex peak waveform judging unit judges whether the noise peaks other than the stutter peaks and the +A peaks are generated in the detection signals of the PCR amplification products based on whether a repeat sequence with at least one nucleotide as a unit other than the microsatellite contained in the DNA fragment is present, and
wherein the peak discrimination processing unit excludes peaks presumed to be the noise peaks other than the stutter peaks and the +A peaks from the fitting targets of the basic waveform by making the distance between a first fitting position of the basic waveform and a second fitting position of the basic waveform separated by more than the unit length of the microsatellite contained in the DNA fragment when the first fitting of the basic waveform to the detection signals of the PCR amplification products is further followed by the second fitting of the basic waveform to these signals for determining whether the DNA fragment is homozygote or heterozygote.

2. The display apparatus according claim 2, wherein a user condition-setting unit is further provided to allow a user to set a nucleotide length of the repeat unit and a threshold of the number of repeats with respect to the repeat sequence other than the microsatellite as a condition of judgment in the complex peak waveform judging unit.

3. The display apparatus according to any one of claims 1 or 2, wherein the display processing unit displays not only a graph of the detection signals of the PCR amplification products, sequence information of the DNA fragment, and a judgment result by the complex peak waveform judging unit but also the discrimination result of the true peaks by the peak discrimination processing unit.

4. A computer-implemented method for discriminating true peaks from noise peaks in waveform data of a fluorescence analysis result obtained from an electrophoresis experiment of PCR amplification products of a DNA fragment containing a microsatellite and displaying the results, comprising the steps of:
judging a complex peak waveform to judge whether or not noise peaks other than stutter peaks with increased or decreased repeat units of the microsatellite in the DNA fragment corresponding to detection signals of the PCR amplification products and +A peaks with one adenine added to the DNA fragment corresponding to the detection signals of the PCR amplification products are generated in the detection signals of the PCR amplification products based on sequence information of the DNA fragment;
processing peak discrimination to discriminate true peaks corresponding to the detection signals of the PCR amplification products of the DNA fragment by fitting a basic waveform,
in which a pattern of appearance of stutter peaks and +A peaks in the detection signals of the PCR amplification products of the DNA fragment is made in a respective model for every kind of the DNA fragment, to the detection signals of the PCR amplification products; and
displaying a discrimination result of true peaks in the peak discrimination processing step, wherein, in the peak discrimination processing step, peaks presumed to be noise peaks other than the stutter peaks and the +A peaks are excluded from fitting targets of the basic waveform when the noise peaks other than the stutter peaks and the +A peaks are judged to be generated in the detection signals of the PCR amplification products in the complex peak waveform judging step,
wherein, in the step of judging a complex peak waveform, whether the noise peaks other than the stutter peaks and the +A peaks are generated in the detection signals of the PCR amplification products is judged based on whether a repeat sequence with at least one nucleotide as a unit other than the microsatellite contained in the DNA fragment is present, and
wherein, in the step of peak discrimination processing, peaks presumed to be the noise peaks other than the stutter peaks and the +A peaks are excluded from the fitting targets of the basic waveform by making the distance between a first fitting position of the basic waveform and a second fitting position of the basic waveform separated by more than the unit length of the microsatellite contained in the DNA fragment when the first fitting of the basic waveform to the detection signals of the PCR amplification products is further followed by the second fitting of the basic waveform to these signals for determining whether the DNA fragment is homozygote or heterozygote.

5. The display method according claim 4, wherein a user condition-setting step is further provided to allow a user to set a nucleotide length of the repeat unit and a threshold of the number of repeats with respect to the repeat sequence other than the microsatellite as a condition of judgment in the step of judging a complex peak waveform.

6. The display method according to any one of claims 4 or 5, wherein, in the step of processing display, not only a graph of the detection signals of the PCR amplification products, sequence information of the DNA fragment, and a judgment result by the complex peak waveform judging unit but also the discrimination result of the true peaks in the peak discrimination processing step is displayed.

7. A computer program product to execute the display method according to any one of claims 4 to 6 on a display apparatus.

## Patentansprüche

1. Anzeigevorrichtung zum Unterscheiden von echten Spitzen von Rausch-Spitzen in Wellenform-Daten eines Fluoreszenzanalyse-Ergebnisses, welches in einem Elektrophorese-Element von PCR-Verstärkungsprodukten eines einen Mikrosatellit enthaltenden DNA-Fragmentes erhalten wurde, und zum Anzeigen der Ergebnisse, wobei die Anzeigevorrichtung Folgendes umfasst:
eine Komplexe-Spitzen-Wellenform-Beurteilungseinheit, die beurteilt, ob solche Rausch-Spitzen in den Detektionssignalen der PCR-Verstärkungsprodukte erzeugt werden, die von Stotter-Spitzen (stutter-peaks) verschieden sind, die erhöhte oder verringerte Wiederholungseinheiten der Mikrosatelliten in dem den Detektionssignalen der PCR-Verstärkungsprodukte entsprechenden DNA-Fragment aufweisen, und von +A-Spitzen verschieden sind, bei denen dem DNA-Fragment, welches den Detektionssignalen der PCR-Verstärkungsprodukte entspricht, ein Adenin zugefügt wurde basierend auf Sequenzinformation des DNA-Fragments;
eine Spitzen-Unterscheidungs-Verbeitungseinheit, welche wahre Spitzen, die den Detektionssignalen der PCR-Verstärkungsprodukte des DNA-Fragments entsprechen, unterscheidet, indem eine Basis-Wellenform an die Detektionssignale der PCR-Verstärkungsprodukte gefittet wird, wobei in der Basiswellenform ein Muster des Auftretens von Stotter-Spitzen und +A-Spitzen in den Detektionssignalen der PCR-Verstärkungsprodukte des DNA-Fragments in einem zugehörigen Modell für jede Art von DNA-Fragment erzeugt ist;
eine Anzeige-Verarbeitungseinheit, die ein Unterscheidungsergebnis der wahren Spitzen durch die Spitzen-Unterscheidungs-Verarbeitungseinheit anzeigt,
wobei die Spitzen-Unterscheidungs-Verarbeitungseinheit Spitzen von dem Fit-Target der Grund-Wellenform ausschließt, von denen angenommen wird, dass sie andere Rausch-Spitzen sind als Stotter-Spitzen und +A-Spitzen, wenn die Komplexe-Spitzen-Wellenform-Beurteilungseinheit die Erzeugung von anderen Rausch-Spitzen als Stotter-Spitzen und +A-Spitzen in den Detektionssignalen der PCR-Verstärkungsprodukte beurteilt,
wobei die Komplexe-Spitzen-Wellenform-Beurteilungseinheit das Urteil, ob die Rausch-Spitzen, die von den Stotter-Spitzen und den +A-Spitzen verschieden sind, in den Detektionssignalen der PCR-Verstärkungsprodukte erzeugt werden basierend darauf fällt, ob eine Wiederholungssequenz mit mindestens einem Nukleotid als Einheit, die von dem in dem DNA-Fragment enthaltenen Mikrosatelliten verschieden ist, vorliegt, und
wobei die Spitzen-Unterscheidungs-Verarbeitungseinheit Spitzen, von denen angenommen wird, dass sie andere Rausch-Spitzen als Stotter-Spitzen und den +A-Spitzen sind, von den Fitting-Targets der Basis-Wellenform ausschließt, indem der Abstand zwischen einer ersten Fit-Position der Basis-Wellenform und einer zweiten Fit-Position der Basis-Wellenform größer gewählt wird als die Einheitslänge des in dem DNA-Fragment enthaltenen Mikrosatelliten, wenn das erste Fitting der Basis-Wellenform an die Detektionssignale der PCR-Verstärkungsprodukte von dem zweiten Fitting der Basis-Wellenform an diese Signale gefolgt wird, um festzustellen, ob das DNA-Fragment homozygot oder heterozygot ist.

2. Anzeigevorrichtung nach Anspruch 1, bei der ferner eine Nutzerbedingungen-Einstelleinheit vorgesehen ist, um es einem Nutzer zu gestatten, eine Nukleotidlänge der Wiederholungseinheit und einen Schwellenwert für die Anzahl von Wiederholungen in Bezug auf die von dem Mikrosatelliten verschiedene Wiederholungssequenz als eine Bedienung für das Urteil in der Komplexe-Spitzen-Wellenform-Beurteilungseinheit einzustellen.

3. Anzeigevorrichtung nach einem der Ansprüche 1 oder 2, bei der die Display-Verarbeitungseinheit nicht nur einen Graph der Detektionssignale der PCR-Verstärkungsprodukte, Sequenzinformation des DNA-Fragments und ein Beurteilungsergebnis durch die Komplexe-Spitzen-Wellenform-Beurteilungseinheit anzeigt, sondern auch das Unterscheidungsergebnis der echten Spitzen durch die Spitzen-Unterscheidungs-Verarbeitungseinheit.

4. Computer-implementiertes Verfahren zum Unterscheiden von echten Spitzen von Rausch-Spitzen in Wellenformdaten eines Fluoreszenz-Analyseergebnisses, welches aus einem Elektrophorese-Experiment von PCR-Verstärkungsprodukten eines einen Mikrosatellit enthaltenden DNA-Fragments erhalten wurde, und zum Anzeigen der Ergebnisse, wobei das Verfahren die folgenden Schritte umfasst:
Beurteilen einer komplexen Spitzen-Wellenform um zu beurteilen, ob solche Rausch-Spitzen in den Detektionssignalen der PCR-Verstärkungsprodukte erzeugt werden, die von Stotter-Spitzen verschieden sind, bei denen erhöhte oder verringerte Wiederholungseinheiten der Mikrosatelliten in dem DNA-Fragment, welches den Detektionssignalen der PCR-Verstärkungsprodukte entspricht, vorliegen, und von +A-Spitzen verschieden sind, bei denen ein Adenin zu dem DNA-Fragment zugefügt ist, welches den Detektionssignalen der PCR-Verstärkungsprodukte entspricht, basierend auf Sequenzinformation des DNA-Fragments;
Verarbeiten einer Spitzen-Unterscheidung, um echte Spitzen zu unterscheiden, die den Detektionssignalen der PCR-Verstärkungsprodukte des DNA-Fragments entsprechen, indem eine Basis-Wellenform mit den Detektionssignalen der PCR-Verstärkungsprodukte gefittet wird, wobei in der Basis-Wellenform ein Muster des Auftretens von Stotter-Spitzen und +A-Spitzen in den Detektionssignalen der PCR-Verstärkungsprodukte des DNA-Fragments in einem zugehörigen Modell für jede Art von DNA-Fragment erzeugt ist; und
Anzeigen eines Unterscheidungsergebnisses der echten Spitzen in dem Spitzen-Unterscheidungs-Verarbeitungsschritt, wobei in dem Spitzen-Unterscheidungs-Verarbeitungsschritt Spitzen, von denen angenommen wird, dass sie Rausch-Spitzen sind, die von Stotter-Spitzen und +A-Spitzen verschieden sind, von den Fit-Targets der Basis-Wellenform ausgeschlossen werden, wenn in dem Komplexe-Spitzen-Wellenform-Beurteilungsschritt beurteilt wird, dass die Rausch-Spitzen, die von den Stotter-Spitzen und den +A-Spitzen verschieden sind, in den Detektionssignalen der PCR-Verstärkungsprodukte erzeugt werden,
wobei in dem Schritt des Beurteilens einer komplexen Spitzen-Wellenform das Urteil, ob die Rausch-Spitzen, die von den Stotter-Spitzen und den +A-Spitzen verschieden sind, in den Detektionssignalen der PCR-Verstärkungsprodukte erzeugt werden, basierend darauf gefällt wird, ob eine Wiederholungssequenz mit mindestens einem Nukleotid als eine Einheit, die von dem in dem DNA-Fragment enthaltenen Mikrosatellit verschieden ist, vorliegt, und
wobei in dem Schritt der Verarbeitung der Spitzen-Unterscheidung Spitzen, von denen angenommen wird, dass sie andere Rausch-Spitzen als Stotter-Spitzen und +A-Spitzen sind, aus den Fit-Targets der Basis-Wellenform ausgeschlossen werden, indem der Abstand zwischen einer ersten Fit-Position der Basis-Wellenform und einer zweiten Fit-Position der Basis-Wellenform größer als eine Einheitslänge des in dem DNA-Fragment enthaltenen Mikrosatelliten gewählt wird, wenn der erste Fit der Basis-Wellenform an die Detektionssignale der PCR-Verstärkungsprodukte ferner von einem zweiten Fit der Basis-Wellenform an diese Signale gefolgt wird, um festzustellen, ob das DNA-Fragment homozygot oder heterozygot ist.

5. Anzeigeverfahren nach Anspruch 4, bei dem ferner ein Schritt zum Einstellen von Nutzerbedingungen vorgesehen ist, der es einem Nutzer gestattet, eine Nukleotidlänge der Wiederholungseinheit und einen Schwellenwert für die Anzahl von Wiederholungen in Bezug auf die Wiederholungssequenz, die von dem Mikrosatellit verschieden ist, als eine Bedingung für die Beurteilung in dem Schritt des Beurteilens einer komplexen Spitzen-Wellenform einzustellen.

6. Anzeigeverfahren nach einem der Ansprüche 4 oder 5, bei dem in dem Schritt des Verarbeitens der Anzeige nicht nur ein Graph der Detektionssignale der PCR-Verstärkungsprodukte, Sequenzinformationen des DNA-Fragments und ein Beurteilungsergebnis der Komplexe-Spitzen-Wellenform-Beurteilungseinheit angezeigt werden, sondern auch das Unterscheidungsergebnis der echten Spitzen in dem Spitzen-Unterscheidungs-Verarbeitungsschritt.

7. Computerprogrammprodukt zum Ausführen des Anzeigeverfahrens nach einem der Ansprüche 4 bis 6 auf einem Anzeigegerät.

## Revendications

1. Dispositif d'affichage pour faire une distinction entre des vrais pics et des pics de bruit dans des données de forme d'onde d'un résultat d'analyse par fluorescence obtenu par électrophorèse de produits d'amplification PCR d'un fragment d'ADN contenant un microsatellite, et afficher le résultat, le dispositif d'affichage comprenant :
une unité d'évaluation de forme d'onde à pics complexes qui évalue si des pics de bruit, autres que des pics de bégaiement avec des unités de répétition augmentées ou diminuées du microsatellite dans le fragment d'ADN correspondant aux signaux de détection des produits d'amplification PCR, et des pics +A avec une adénine ajoutée au fragment d'ADN correspondant aux signaux de détection des produits d'amplification PCR, sont générés ou pas dans les signaux de détection des produits d'amplification PCR sur la base d'une information de séquence du fragment d'ADN ;
une unité de traitement de distinction de pics qui distingue des vrais pics correspondant aux signaux de détection des produits d'amplification PCR du fragment d'ADN par concordance d'une forme d'onde basique, dans laquelle un motif d'apparence de pics de bégaiement et de pics +A dans les signaux de détection des produits d'amplification PCR du fragment d'ADN est fait selon un modèle respectif pour chaque sorte de fragment d'ADN, par rapport à des signaux de détection des produits d'amplification PCR ;
une unité de traitement d'affichage qui affiche un résultat de distinction des vrais pics par l'unité de traitement de distinction de pics,
dans lequel l'unité de traitement de distinction de pics exclue des pics présumés être des pics de bruit, autres que des pics de bégaiement et des pics +A, de la concordance avec des cibles de la forme d'onde basique lorsque l'unité d'évaluation de forme d'onde à pics complexes évalue une génération des pics de bruit autres que les pics de bégaiement et les pics +A dans les signaux de détection des produits d'amplification PCR,
dans lequel l'unité d'évaluation de forme d'onde à pics complexes évalue si les pics de bruit autres que les pics de bégaiement et les pics +A sont générés dans les signaux de détection des produits d'amplification PCR sur la base de la présence d'une séquence de répétition avec au moins un nucléotide en tant qu'unité différente du microsatellite contenu dans le fragment d'ADN, et
dans lequel l'unité de traitement de distinction de pics exclue des pics présumés être des pics de bruit autres que les pics de bégaiement et les pics +A de la concordance avec des cibles de la forme d'onde basique, en faisant en sorte que la distance entre une première position de concordance de la forme d'onde basique et une deuxième position de concordance de la forme d'onde basique soit supérieure à la longueur unitaire du microsatellite contenu dans le fragment d'ADN lorsque la première concordance de la forme d'onde basique par rapport aux signaux de détection des produits d'amplification PCR est en outre suivie d'une deuxième concordance de la forme d'onde basique par rapport à ces signaux pour déterminer si le fragment d'ADN est homozygote ou hétérozygote.

2. Dispositif d'affichage selon la revendication 1, dans lequel une unité d'établissement de conditions d'utilisateur est en outre prévue pour permettre qu'un utilisateur règle une longueur de nucléotide de l'unité de répétition et un seuil du nombre de répétitions en ce qui concerne la séquence de répétition autre que le microsatellite, en tant que condition d'évaluation dans l'unité d'évaluation de forme d'onde à pics complexes.

3. Dispositif d'affichage selon l'une quelconque des revendications 1 ou 2, dans lequel l'unité de traitement d'affichage affiche non seulement un graphe des signaux de détection des produits d'amplification PCR, des informations de séquence du fragment d'ADN, et un résultat d'évaluation par l'unité d'évaluation de forme d'onde à pics complexes, mais aussi les résultats de distinction des vrais pics par l'unité de traitement de distinction de pics.

4. Procédé mis en oeuvre par un ordinateur pour faire une distinction entre des vrais pics et des pics de bruit dans des données de forme d'onde d'un résultat d'analyse par fluorescence obtenu par électrophorèse de produits d'amplification PCR d'un fragment d'ADN contenant un microsatellite, et afficher les résultats, comprenant les étapes suivantes :
évaluer une forme d'onde à pics complexes pour évaluer si des pics de bruit, autres que des pics de bégaiement avec des unités de répétition augmentées ou diminuées du microsatellite dans le fragment d'ADN correspondant à des signaux de détection des produits d'amplification PCR et des pics +A avec une adénine ajoutée au fragment d'ADN correspondant aux signaux de détection des produits d'amplification PCR, sont générés dans les signaux de détection des produits d'amplification PCR sur la base d'informations de séquence du fragment d'ADN ;
faire un traitement de distinction de pics pour distinguer des vrais pics correspondant aux signaux de détection des produits d'amplification PCR du fragment d'ADN, par concordance d'une forme d'onde basique, dans laquelle un motif d'apparence de pics de bégaiement et de pics +A dans les signaux de détection des produits d'amplification PCR du fragment d'ADN est fait selon un modèle respectif pour chaque sorte de fragment d'ADN, par rapport aux signaux de détection des produits d'amplification PCR ; et
afficher un résultat de distinction de vrais pics dans l'étape de traitement de distinction de pics,
dans lequel dans l'étape de traitement de distinction de pics, des pics présumés être des pics de bruit autres que les pics de bégaiement et les pic +A sont exclus de la concordance avec des cibles de la forme d'onde basique lorsque les pics de bruit autres que les pics de bégaiement et les pics +A sont évalués comme étant générés dans les signaux de détection des produits d'amplification PCR dans l'étape d'évaluation d'une forme d'onde à pics complexes,
dans lequel, dans l'étape d'évaluation d'une forme d'onde à pics complexes, l'évaluation si les pics de bruit autres que les pics de bégaiement et les pics +A sont générés dans les signaux de détection des produits d'amplification PCR, est faite sur la base de la présence d'une séquence de répétition avec au moins un nucléotide en tant qu'unité autre que le microsatellite contenu dans le fragment d'ADN, et
dans lequel, dans l'étape de traitement de distinction de pics, des pics présumés être des pics de bruit autres que les pics de bégaiement et les pics +A, sont exclus de la concordance avec des cibles de la forme d'onde basique en faisant en sorte que la distance entre une première position de concordance de la forme d'onde basique et une deuxième position de concordance de la forme d'onde basique soit supérieure à la longueur unitaire du microsatellite contenu dans le fragment d'ADN lorsque la première concordance de la forme d'onde basique par rapport aux signaux de détection des produits d'amplification PCR est en outre suivie par la deuxième concordance de la forme d'onde basique par rapport à ces signaux pour déterminer si le fragment d'ADN est homozygote ou hétérozygote.

5. Procédé d'affichage selon la revendication 4, dans lequel une étape d'établissement de conditions d'utilisateur est en outre prévue pour permettre qu'un utilisateur règle une longueur de nucléotide de l'unité de répétition et un seuil du nombre de répétitions en ce qui concerne la séquence de répétition autre que le microsatellite, en tant que condition d'évaluation dans l'étape d'évaluation d'une forme d'onde à pics complexes.

6. Procédé d'affichage selon l'une quelconque des revendications 4 ou 5, dans lequel, dans l'étape de traitement d'affichage, sont affichés non seulement un graphe des signaux de détection des produits d'amplification PCR, des informations de séquence du fragment d'ADN et un résultat d'évaluation par l'unité d'évaluation de forme d'onde à pics complexes, mais aussi les résultats de distinction des vrais pics dans l'étape de traitement de distinction de pics.

7. Produit programme d'ordinateur pour exécuter le procédé d'affichage selon l'une quelconque des revendications 4 à 6 sur un dispositif d'affichage.
